# EUROPEAN PATENT SPECIFICATION

(11) **EP 3 420 348 B1**
(45) Date of publication and mention of the grant of the patent: **07.06.2023**
(21) Application number: 17757151.0
(22) Date of filing: 22.02.2017
(51) Int. Cl.: C12Q 1/6883, G01N 33/00, G01N 33/48, G01N 33/483

(54) **METHOD OF DETERMINING THE RISK OF DEVELOPING ACUTE KIDNEY INJURY**
VERFAHREN ZUR BESTIMMUNG DES RISIKOS DER ENTWICKLUNG VON AKUTER NIERENLÄSION
MÉTHODE DE DÉTERMINATION DU RISQUE DE DÉVELOPPER UNE INSUFFISANCE RÉNALE AIGUË

(30) Priority: 22.02.2016 US 201662298394 P
(43) Date of publication of application: 02.01.2019
(73) Proprietor: Sapere Bio, Inc., Research Triangle Park, NC 27709 (US)
(72) Inventor: MITIN, Natalia, Durham NC 27713 (US)
(74) Representative: Hoffmann Eitle
(86) International application number: PCT/US2017/018962
(87) International publication number: WO 2017/147195

(56) References cited:
- WO-A2-01/40300
- WO-A9-2010/091236
- US-A1- 2005 272 038
- US-A1- 2008 108 062
- US-A1- 2013 122 530
- US-A1- 2013 225 432
- US-A1- 2015 218 240
- ZHU-ZHI WEN ET AL: "Angiotensin II Receptor Blocker Attenuates Intrarenal Renin-Angiotensin-System and Podocyte Injury in Rats with Myocardial Infarction", PLOS ONE, vol. 8, no. 6, 14 June 2013 (2013-06-14), pages 1-11, XP055413103, DOI: 10.1371/journal.pone.0067242
- DAVID H. LEE ET AL: "INK4a deletion results in improved kidney regeneration and decreased capillary rarefaction after ischemia-reperfusion injury", AMERICAN JOURNAL OF PHYSIOLOGY: RENAL PHYSIOLOGY, vol. 302, no. 1, 1 January 2012 (2012-01-01), pages F183-F191, XP055614500, United States ISSN: 1931-857X, DOI: 10.1152/ajprenal.00407.2011
- S. ANDERSON ET AL: "Acute Kidney Injury in Older Adults", JOURNAL OF THE AMERICAN SOCIETY OF NEPHROLOGY., vol. 22, no. 1, 1 January 2011 (2011-01-01), pages 28-38, XP055413112, US ISSN: 1046-6673, DOI: 10.1681/ASN.2010090934
- WEN, Z ET AL.: 'Angiotensin II Receptor Blocker Attenuates Intrarenal Renin-Angiotensin-System and Podocyte Injury in Rats with Myocardial Infarction' PLOS ONE vol. 8, no. 6, 2013, pages E67242-1 - E67242-11, XP055413103
- DATABASE GENBANK 01 December 2012 'Homo Sapiens Isolate Ipch460_Cols Cyclindependent Kinase Inhibitor 2A (CDKN2A) mRNA, Complete Cds', XP055413107 Database accession no. JQ694043
- ANDERSON, S ET AL.: 'Acute Kidney Injury In Older Adults' JOURNAL OF THE AMERICAN SOCIETY OF NEPHROLOGY vol. 22, no. 1, 2011, pages 28 - 38, XP055413112

## Description

### TECHNICAL FIELD

Described herein are methods, kits, and compositions of matter for identifying subjects that are at risk for developing acute kidney injury. These methods, kits, and compositions are useful for identifying subjects at risk for developing acute kidney injury prior to undergoing a surgical intervention, such as cardiovascular surgery, or prior to initiating chemotherapy. Also, described herein are non-naturally occurring DNA sequences that are useful in identifying subjects at risk for developing acute kidney injury.

### BACKGROUND OF THE INVENTION

Acute kidney injury (AKI) is the transient loss of kidney function due to ischemia, inflammatory disease or nephrotoxicity. The pathogenesis of AKI is complex and can include a number of haemodynamic, inflammatory, metabolic and nephrotoxic factors. Hospital acquired AKI is commonly caused by cardiovascular surgery and invasive coronary angiography. The incidence of AKI can be as high as 30% in cardiac intervention patients and its development is independently associated with an increased risk of in-hospital death. It usually takes 2-3 days post procedure for the AKI to become apparent and it is characterized by an increase in absolute serum creatinine (SCr) levels of at least 0.3 mg/dL or a 50% relative increase over baseline levels. Mortality rates associated with AKI can exceed 50% in some patient populations and remain high despite advances in supportive care. Although only approximately 1% of AKI patients experience SCr increases of up to 300% and progress to end stage renal disease, even small increases in serum creatinine (50%) are associated with a significant increase in 30-day mortality, prolonged hospital stays, and long-term adverse cardiac and renal events, making AKI both dangerous and cost intensive.

Although there is no generally accepted diagnostic for identifying patients at high risk for developing AKI, several clinical markers and patient characteristics have been identified as being associated with a patient's increased risk for developing AKI. In general, patients with preexisting renal insufficiency or diabetes are at higher risk for developing AKI. However, regardless of baseline renal function, patients who develop AKI are at an increased risk for complications as compared to patients who do not develop AKI.

Traditionally, physicians measure creatinine to check renal function. Creatinine levels are used to calculate estimated glomerular filtration rate (eGFR) using serum creatinine, age, and gender. Patients with eGFR >60 mL/min/1.73 m² are considered to have normal kidney function whereas patients with eGFR between 59 and 15 mL/min/1.73 m² may have kidney disease.

Although creatinine/eGFR remains the most widely measured indicator of renal function, GFR estimates remain relatively imprecise especially in elderly patients and patients that exhibit renal injury or have a body mass index (BMI) outside of the range used to calculate eGFR. In addition, AKI diagnosis in hospitalized patients can be achieved by serial monitoring of creatinine levels throughout a patient's hospital stay. However, changes in creatinine are not typically detectable until 2-3 days after kidney injury occurs, at which point about 50% of kidney function has already been lost. Thus, measurement of creatinine levels is generally an ineffective strategy for early detection of AKI in a hospital setting and especially for detecting hospital acquired AKI in patients undergoing outpatient procedures.

Outpatient catheterization procedures for the diagnosis and treatment of many cardiovascular disorders are increasingly popular with patients and third party payers since they are often associated with actual or perceived reduced costs risks. However, all procedures have risks. While patients undergoing cardiac surgery have significantly higher in-hospital mortality rates (within 30-days) as compared to patients undergoing angioplasty, patients undergoing angioplasty have significantly higher rates of repeat revascularization procedures as compared to cardiac surgery patients. Catheterization patients are also susceptible to contrast-induced AKI (CI-AKI) or contrast-induced nephropathy (CIN), an acute deterioration of renal function after administration of contrast media frequently used in cardiac catheterization procedures. Patients experiencing CI-AKI generally have more complications, prolonged hospital stay, and adverse long-term outcomes including morbidity than patients without CI-AKI. In addition, cardiac surgery in patients with severe, multi-vessel disease seems to provide a survival advantage over angioplasty, the advantage being seen as early as 2.5 years after the procedure. Therefore, both catheterization and open surgical cardiac procedures carry risk of hospital induced AKI.

The early diagnosis of AKI has been problematic owing to the absence of tests with sufficient accuracy that can be performed early in the course of injury and predict subsequent loss of kidney function. In response to this problem, several biomarkers have been recently investigated as possible tools for the early detection of AKI. Among these biomarkers, particularly promising results have been reported for neutrophil gelatinase-associated lipocalin (NGAL), cystatin C, and a recently approved TIMP-2/IGFBP7 biomarker panel collectively named NephroCheck. For example, urine NGAL appears to be of value in the prediction of occurrence, duration and severity of AKI after pediatric cardiac surgery and serum cystatin C is predictive of AKI 1 to 2 days earlier than serum creatinine in critically ill patients. Also, serum cystatin C appears to be superior to serum creatinine for detecting chronic kidney disease and, unlike creatinine, cystatin C is not affected by body mass index, diet, or drugs. Nephrocheck can predict risk of developing AKI in patients admitted to the ICU within 12h of admission; however, it has a 50% false positive rate.

A number of prophylaxes are available as measures to prevent or reduce the development of AKI in cardiovascular surgical patients. However, these measures are not without complication. For example, intravenous volume expansion with a saline solution prior to and during cardiac procedure and administration of a limited volume of contrast medium provide significant benefit towards AKI prevention in high risk patients. However, such procedures may cause complications such as electrolyte imbalances, gastrointestinal distress and hypertension and, therefore, are not appropriate for widespread use in low risk patients.

Thus, there is a need for additional biomarkers that can be used to identify patients prior to cardiovascular surgical intervention that are at high risk for developing AKI after a cardiovascular procedure. Such biomarkers would allow clinicians to target these patients with renal protective strategies, including pre-operative, intra-operative, and post-operative measures, and improve patient outcomes.

US 2013/122530 A1 concerns prediction and recognition of acute kidney injury after surgery.

David H. Lee et al., American Journal of Physiology: Renal Physiology, vol. 302, no. 1, 1 January 2012 (2012-01-01), pages F183-F191, concerns INK4a deletion results in improved kidney regeneration and decreased capillary rarefaction after ischemia-reperfusion injury.

### BRIEF SUMMARY OF THE INVENTION

The present invention is defined by the claims. Described herein are methods, kits, and compositions of matter for identifying subjects that are at risk for developing acute kidney injury. In some embodiments described herein the levels of p14^{ARF} and p16^{INK4a} are measured in a patient sample. Levels of these biomarkers beyond a pre-determined threshold correspond with the risk of developing acute kidney injury. The levels of p14^{ARF} and p16^{INK4a} may be measured by any suitable means described herein. In some embodiments, the risk for developing acute kidney injury is determined by measuring the levels of a non-naturally occurring DNA sequences.

One embodiment described herein is a non-naturally occurring DNA sequence comprising a first DNA sequence segment having 88% identity to SEQ ID NO: 1 connected by a phosphodiester linkage to a second DNA sequence segment comprising between 5 and 316 bases having at least an 85% identity to any one of SEQ ID NO: 2-5.

Another embodiment described herein is a method of generating a non-naturally occurring DNA sequence from a subject prior to cardiovascular surgical intervention comprising obtaining a blood sample from the subject and generating the non-naturally occurring DNA sequence from the blood sample; wherein the sequence comprises a first DNA sequence segment having 88% identity to SEQ ID NO: 1 connected by a phosphodiester linkage to a second DNA sequence segment comprising between 5 and 316 bases having 85% identity to any one of SEQ ID NO: 2-5. In another aspect, the method further comprises isolating peripheral blood T lymphocytes from the blood sample. In one aspect, the method further comprises extracting RNA from the blood sample and generating a cDNA library. In another aspect, the method further comprises amplifying the non-naturally occurring DNA sequence from the cDNA library by a polymerase chain reaction. In another aspect, the amplification comprises one or more primers and probes according to any one of SEQ ID NO: 8-13 or 16-18. In another aspect, the method further comprises extracting RNA from the blood sample and generating a cDNA library. In another aspect, the method further comprises amplifying the non-naturally occurring DNA sequence from the cDNA library by a polymerase chain reaction.

Another embodiment described herein, the method of generating a non-naturally occurring DNA sequence further comprises detecting a level of the non-naturally occurring DNA sequence. In another aspect, the method further comprises identifying a subject as being at risk of acute kidney injury when a level of the non-naturally occurring DNA sequence is equal to or higher than a pre-determined threshold level of the non-naturally occurring DNA sequence, wherein levels above the threshold is indicative of acute kidney injury.

Another embodiment described herein is a method for identifying risk of acute kidney injury in subjects prior to cardiovascular surgical intervention comprising: a) obtaining a blood sample from a subject prior to the cardiovascular surgical intervention; b) generating a non-naturally occurring DNA sequence from the blood sample, wherein the sequence comprises a first DNA sequence segment having 88% identity to SEQ ID NO: 1 connected by a phosphodiester linkage to a second DNA sequence segment comprising between 5 and 316 bases having 85% identity to one or more of SEQ ID NO: 2-5; and c) detecting a level of the non-naturally occurring DNA sequence. In one aspect, the method further comprises isolating peripheral blood T lymphocytes from the blood sample of step (a). In another aspect, the method further comprises: d) comparing the level of the non-naturally occurring DNA sequence to a pre-determined threshold level of the non-naturally occurring DNA sequence; and e) identifying the subject as being at risk of acute kidney injury when the level of the non-naturally occurring DNA sequence is equal to or higher than a pre-determined threshold level of the non-naturally occurring DNA sequence.

Another embodiment described herein is a method for identifying risk of acute kidney injury in subjects prior to cardiovascular surgical intervention comprising: a) obtaining a blood sample from a subject prior to the subject undergoing cardiovascular surgical intervention; b) detecting a level of gene expression of p14^{ARF} and p16^{INK4a} in the sample; c) comparing the level of gene expression of p14^{ARF} and p16^{INK4a} in the sample to a pre-determined threshold level of gene expression; and d) identifying the subject as being at risk of acute kidney injury when the gene expression level of at least one of p14^{ARF} and p16^{INK4a} is equal to or higher than the pre-determined threshold level of gene expression. In one aspect, the method further comprises isolating peripheral blood T lymphocytes from the blood sample of step (a), and detecting the level of expression of p14^{ARF} and p16^{INK4a} in the peripheral blood T lymphocytes from the sample. In another aspect, the level of gene expression is detected by a method comprising quantitative PCR, microarray, or RNA sequencing. In another aspect, the level of gene expression is detected by detecting a gene product of p14^{ARF} and p16^{INK4a} in the sample. In another aspect, the gene product is detected by a method comprising solid phase immunoassays, western blotting, or 2-D gel separation. In another aspect, the pre-determined threshold is determined by ROC analysis.

In some embodiments, the cardiovascular surgical intervention is coronary artery bypass surgery. In some other aspects, the cardiovascular surgical intervention is a cardiac catheterization procedure.

In some embodiments, the non-naturally occurring DNA sequence described herein is connected by a phosphodiester linkage to a third DNA sequence segment comprising between 5 and 316 bases having 85% identity to any one of SEQ ID NO: 2-5. In some embodiments, the non-naturally occurring DNA sequence comprises SEQ ID NO: 2, SEQ ID NO: 1, and SEQ ID NO: 4 in a 5' to 3' direction. In some embodiments, the non-naturally occurring DNA sequence comprises SEQ ID NO: 3, SEQ ID NO: 1, and SEQ ID NO: 4 in a 5' to 3' direction.

In some embodiments, the non-naturally occurring DNA sequence comprises a total combined sequence length of between 30 and 150 bases. In some aspects, the non-naturally occurring DNA sequence comprises a total combined sequence length of between 30 and 75 bases. In some aspects, the non-naturally occurring DNA sequence comprises a total combined sequence length of between 30 and 50 bases. In some aspects, the non-naturally occurring DNA sequence comprises one or more non-naturally occurring bases.

In some embodiments, the non-naturally occurring DNA sequence is SEQ ID NO: 6.

In some embodiments, the non-naturally occurring DNA sequence is SEQ ID NO: 7.

In some embodiments, the non-naturally occurring DNA sequence is SEQ ID NO: 25.

In some embodiments described herein, the methods for identifying risk of acute kidney injury in subjects prior to cardiovascular surgical intervention comprises a sensitivity of about 30% to about 100%. In some aspects, the identifying comprises a sensitivity of about 50% to about 100%. In some aspects, the identifying comprises a sensitivity of about 70% to about 100%.

In some embodiments described herein, the methods for identifying risk of acute kidney injury in subjects prior to cardiovascular surgical intervention comprises a specificity of about 30% to about 100%. In some aspects, the identifying comprises a specificity of about 50% to about 100%. In some aspects, the identifying comprises a specificity of about 70% to about 100%.

The references to methods of treatment in the present disclosure are to be interpreted as references to the compositions of the present invention for use in a method of treatment of the human (or animal) body by therapy (or for diagnosis).

Another instance described herein is a method for treating acute kidney injury in a cardiac surgical subject comprising identifying a subject at risk for acute kidney injury as by the methods described herein and treating the subject prior to cardiac surgery by administering to the subject one or more prophylactic treatments for acute kidney injury.

Another instance described herein is a method for treating acute kidnev iniurv in a cardiac surgical subject comprising: a) requesting a result of a clinical test, wherein the clinical test comprises the methods of identifying a subject at risk for acute kidney injury as described herein and b) treating the subject prior to cardiac surgery by administering to the subject one or more prophylactic treatments for acute kidney injury.

In some instances described herein, the one or more prophylactic treatments comprises intravenous volume hydration comprising isotonic sodium chloride solution, protocol-based management of hemodynamic parameters comprising administration of fluid, blood products and vasopressors, minimizing amount of contrast media used for diagnosis, avoiding other precipitating factors of AKI comprising discontinuation of ACE inhibitors, NSAIDs and other drugs known to cause kidney injury before surgery, administering pharmaceutically active agents comprising vasodilators, calcium channel blockers, inotropic agents, aminophylline, growth factors, vasoactive peptides, adhesion molecules, or endothelin inhibitors, or a limited use of diuretics comprising loop diuretics.

Another instance described herein is a kit for identifying risk of acute kidney injury in subjects prior to cardiovascular surgical intervention comprising: a) a set of reagents for generating a non-naturally occurring DNA sequence described herein generated by the methods described herein from a biological sample of a subject via quantitative PCR comprising a set of primers and probes; b) reference or control RNA; and c) a set of instructions for determining if a subject is at risk for developing acute kidney injury. In one aspect, the kit comprises a set of primers and probes comprising one or more of SEQ ID NO:8, SEQ ID NO:9, SEQ ID NO:10, SEQ ID NO:11, SEQ ID NO:12, SEQ ID NO:13, SEQ ID NO:16, SEQ ID NO:17, or SEQ ID NO:18 or combinations thereof.

### BRIEF DESCRIPTION OF THE DRAWINGS

Figure 1 represents receiver operating characteristic ("ROC") analysis of p16^{Ink4a} expression levels with respect to the clinical endpoint acute kidney injury (AKI).
Figure 2 represents receiver operating characteristic ("ROC") analysis of p14^{Arf} expression levels with respect to the clinical endpoint acute kidney injury (AKI).
Figure 3 represents receiver operating characteristic ("ROC") analysis of p14^{Arf} expression levels with respect to the clinical endpoint 30 day major adverse clinical events (30d-MACE).
Figure 4 represents receiver operating characteristic ("ROC") analysis of p14^{Arf} expression levels with respect to the clinical endpoint acute kidney injury (AKI).
Figure 5 represents receiver operating characteristic ("ROC") analysis of p14^{Arf} expression levels with respect to the clinical endpoint acute kidney injury (AKI) for patients with the wild-type (Panel A)(AA) or heterozygous (AG) rs10757278 locus.
Figure 6 represents receiver operating characteristic ("ROC") analysis of p14^{Arf} expression levels with respect to the clinical endpoint acute kidney injury (AKI) for patients with homozygous mutation in rs10757278 locus (GG).
Figure 7 represents receiver operating characteristic ("ROC") analysis of p14^{Arf} expression levels with respect to the clinical endpoint acute kidney injury (AKI) for patients with homozygous mutation in rs10757278 locus (GG).

### DETAILED DESCRIPTION OF THE INVENTION

The following paragraphs define in more detail the embodiments of the invention described herein. The following embodiments are not meant to limit the invention or narrow the scope thereof, as it will be readily apparent to one of ordinary skill in the art that suitable modifications and adaptations may be made without departing from the scope of the invention, as defined by the appended claims.

For purposes of interpreting this specification, the following terms and definitions will apply and whenever appropriate, terms used in the singular will also include the plural and vice versa.

As used herein, the phrase "cardiovascular surgical intervention" means one or more invasive procedures affecting the cardiovascular system of a patient. Non-limiting examples are coronary angioplasty, including balloon angioplasty and coronary artery balloon dilation, percutaneous coronary intervention, laser angioplasty, atherectomy, coronary bypass graft surgery (CABG), valve repair, minimally invasive heart surgery including limited access coronary artery surgery, port-access coronary artery bypass (PACAB or PortCAB), and minimally invasive coronary artery bypass graft (MIDCAB), catheter ablation, transmyocardial revascularization, heart transplant, and artificial heart valve surgery.

As used herein, a "subject" can be an individual that is a human or other animal. A "patient" refers to a class of subjects who is under the care of a treating physician (e.g., a medical doctor or veterinarian). The subject can be male or female of any age. Exemplary and non-limiting subjects include, humans, rabbits, mice, rats, horses, dogs, and cats. In one embodiment, the subject has undergone or will undergo a surgical intervention, such as a cardiovascular surgical intervention described herein. In other embodiments, the subject has been treated or will be treated with a chemotherapeutic, for example, cisplatin.

As used herein, the phrase "treatments for acute kidney injury" means administering one or more pharmaceutically active agents, performing one or more procedures, or adding or modifying one or more protocols of a patient's cardiovascular surgical procedure, prior to, during, and/or after the surgical procedure that is known to reduce or prevent the incidence of AKI in a patient undergoing cardiovascular surgical intervention. Such agents, procedures or protocols are known to those skilled in the art. Non-limiting examples include intravenous volume hydration including isotonic sodium chloride solution, bicarbonate buffers, and Plasmalyte, protocol-based management of hemodynamic parameters such as administration of fluid, blood products and vasopressors, minimizing amount of contrast media used for diagnosis, avoiding other precipitating factors of AKI such as discontinuation of ACE inhibitors, NSAIDs and other drugs known to cause kidney injury before surgery, administering pharmaceutically active agents such as vasodilators, calcium channel blockers, inotropic agents, aminophylline, growth factors, vasoactive peptides, adhesion molecules, and endothelin inhibitors, and the careful use of diuretics, specifically loop diuretics.

The term "sample," as used herein, refers to a composition that is obtained or derived from a subject that contains genomic information. The sample can be whole blood or a blood sample that has been fractionated. The sample may be peripheral blood leukocytes including neutrophils, eosinophils, basophils, lymphocytes, and monocytes. In some embodiments, the sample is a peripheral blood lymphocyte selected from B cells, T cells and NK cells. In one embodiment, the sample is a peripheral blood T lymphocyte (e.g., a T cell) or a subset of T cells (e.g., CD3+ cells). In another embodiment, the sample is a tissue biopsy.

As used herein, the term "gene" refers to a nucleic acid that encodes an RNA, for example, nucleic acid sequences including, but not limited to, structural genes encoding a polypeptide. The term "gene" also refers broadly to any segment of DNA associated with a biological function. As such, the term "gene" encompasses sequences including but not limited to a coding sequence, a promoter region, a transcriptional regulatory sequence, a non-expressed DNA segment that is a specific recognition sequence for regulatory proteins, a non-expressed DNA segment that contributes to gene expression, a DNA segment designed to have desired parameters, or combinations thereof. A gene can be obtained by a variety of methods, including cloning from a biological sample, synthesis based on known or predicted sequence information, and recombinant derivation from one or more existing sequences.

The term "gene expression" generally refers to the cellular processes by which a biologically active polypeptide is produced from a DNA sequence and exhibits a biological activity in a cell. As such, gene expression involves the processes of transcription and translation, but also involves post-transcriptional and post-translational processes that can influence a biological activity of a gene or gene product. These processes include, but are not limited to RNA synthesis, processing, and transport, as well as polypeptide synthesis, transport, and post-translational modification of polypeptides. Additionally, processes that affect protein-protein interactions within the cell can also affect gene expression as defined herein. In some embodiments, the phrase "gene expression" refers to a subset of these processes. As such, "gene expression" refers in some embodiments to transcription of a gene in a cell type or tissue. Thus, the phrase "expression level" can refer to a steady state level of an RNA molecule in a cell, the RNA molecule being a transcription product of a gene. Expression levels can be expressed in whatever terms are convenient, and include, but are not limited to absolute and relative measures. For example, an expression level can be expressed as the number of molecules of mRNA transcripts per cell or per microgram of total RNA isolated from cell. Alternatively or in addition, an expression level in a first cell can be stated as a relative amount versus a second cell (e.g., a fold enhancement or fold reduction), wherein the first cell and the second cell are the same cell type from different subjects, different cell types in the same subject, or the same cell type in the same subject but assayed at different times (e.g., before and after a given treatment, at different chronological time points, etc.).

The term "gene product" generally refers to the product of a transcribed gene, such as a protein, peptide, or enzyme. However a gene product may also refer to non-proteins, such as a functional RNA (fRNA), for example, micro RNAs (miRNA), piRNAs, ribosomal RNAs (rRNAs), transfer RNAs (tRNAs), and the like.

The terms "template nucleic acid" and "target nucleic acid" as used herein each refers to nucleic acids isolated from a biological sample as described herein above.

The term "target-specific primer" refers to a primer that hybridizes selectively and predictably to a target sequence, for example a target sequence present in an mRNA transcript derived from a p16INK4a gene or an ARF gene. A target-specific primer can be selected or synthesized to be complementary to known nucleotide sequences of target nucleic acids.

The term "primer" as used herein refers to a contiguous sequence comprising in some embodiments about 6 or more nucleotides, in some embodiments about 10-20 nucleotides (e.g. 15-mer), and in some embodiments about 20-30 nucleotides (e.g. a 22-mer). Primers used to perform the method of the presently disclosed subject matter encompass oligonucleotides of sufficient length and appropriate sequence so as to provide initiation of polymerization on a nucleic acid molecule.

The term "sensitivity" refers to a measurement of the proportion of actual positively identified results in a binary test (e.g., the proportion of individuals identified as having a disease or condition who are correctly identified as having the disease or condition in a diagnostic test).

The term "specificity" refers to a measurement of the proportion of actual negatively identified results in a binary test (e.g., the proportion of individuals identified as not having a disease or condition that are correctly identified as not having the disease or condition in a diagnostic test).

The term "negative predictive value" refers to the proportion of identified negative results that are actually negative for a disease or condition in a diagnostic test.

The term "positive predictive value" refers to the proportion of identified positive results that are actually positive for a disease or condition in a diagnostic test.

The term "threshold" refers to a specific level at which a measured parameter has been established. The exact threshold values and the diagnostic correlations to a disease state, such as AKI vary depending on the gene expression measuring assay and can be determined empirically by comparision to reference samples that have been shown to be positive and negative for acquiring AKI. Expression levels above this threshold and below this threshold are indicative of a positive or negative diagnostic outcome, respectively. A specific cutoff for the threshold may be set depending on the desired sensitivity and specificity for a subject population. In some embodiments described herein, the threshold level of a non-naturally occurring DNA sequence may be used to identify a subject as likely to acquire AKI.

The terms "predicting" and "likelihood" as used herein does not mean that the outcome is occurring with 100% certainty. Instead, it is intended to mean that the outcome is more likely occurring than not. Acts taken to "predict" or "make a prediction" can include the determination of the likelihood that an outcome is more likely occurring than not.

The terms p14^{ARF} refers to the gene encoded by the cyclin dependent kinase inhibitor 2a (CDKN2A) transcript variant 4. This gene corresponds to the National Center for Biotechnology Information (NCBI) accession numbers NM_058195.3 (mRNA) and NP_478102.1 (protein). As used herein, p14^{ARF} refers also to p14 or any other common gene synonym.

The term p16^{INK4a} refers to the gene encoded by the cyclin dependent kinase inhibitor 2a (CDKN2A) transcript variant 1. This gene corresponds to the National Center for Biotechnology Information (NCBI) accession numbers NM_000077.4 (mRNA) and NP_000068.1 (protein). As used herein, p16^{INK4a} refers also to p 16 or any other common gene synonym.

There are several definitions of severity of kidney injury, such as the as Risk, Injury, Failure; and Loss; and End-stage kidney disease (RIFLE) criteria created by the Acute Dialysis Quality Initiative. Risk is defined as 50-99% increase in serum creatinine as compared to baseline; injury is defined as 100-199% increase; failure is defined as >200% increase, and loss is defined as acute renal failure for over 4 weeks that requires dialysis. RIFLE criteria were incorporated into newer guidelines put forth by the Kidney Disease Improving Global Outcomes (KDIGO) in 2012. As used herein, AKI is defined as an increase in serum creatinine by 0.3mg/dL or more within 48 hours or >50% or more within the last 7 days. Severity of AKI is defined by the following stages stage 1- 50-99% increase in serum creatinine as compared to baseline, stage 2- 100-199% increase, and stage 3 >200% increase.

Unless defined otherwise, technical and scientific terms used herein have the same meaning as commonly understood by one of ordinary skill in the art to which this invention belongs.

AKI is associated with a significant increase in 30-day mortality, prolonged hospital stays, and long-term adverse cardiac and renal events. Although supportive care is sometimes effective for treating patients that experience AKI, preventing the occurrence of AKI is substantially more effective that treating AKI once it has developed. For example, patients undergoing cardiovascular surgical intervention experience hospital acquired AKI at an incidence as high as 30%. In addition, AKI is a common side effect of cancer patients recieving a standard chemotherapy regimen. Therefore, identifying patients at risk for developing AKI prior to an invasive surgical procedure or prior to initiating chemotherapy can provide better patient outcomes and decreased healthcare costs. Alternatively, identifying patients at risk for developing AKI soon after surgery or following chemotherapy initiation may allow for a treatment of AKI to be initiated.

As described herein, it was discovered that the INK4a/ARF locus is useful for establishing AKI susceptibility. Specifically, the levels of p14^{ARF} and/or p16^{INK4a} are indicative of AKI susceptibility. In some studies, p14^{ARF} appears to be a more reliable predictor of any incidence of AKI, whereas p16^{INK4a} may be an indicator of the severity of the AKI. The INK4a/ARF locus (also called CDKN2A) on chromosome 9p21 encodes two distinct genes, namely p14^{ARF} and p16^{INK4a}. Changes in expression of these genes has been associated with a variety of human neoplasms. U.S. Patent No. 8,158,347 describes methods for determining the molecular age of a cell or tissue by quantitating expression levels of p14^{ARF} and/or p16^{INK4a} and comparing such levels to certain standards to determine whether the cell or tissue is older, younger, or the same as the chronological age of the cell or tissue.

Despite this understanding, the role of the INK4a/ARF locus in AKI has been, prior to this disclosure, completely unknown. Therefore, determining the levels of these genes may also be useful in identifying patients with dispositions to a variety of diseases, a patient's suitability for organ transplant, or for predicting therapy-induced toxicity such as toxicity caused by chemotherapy or radiotherapy.

Therefore, the present invention provides methods for identifying risk of AKI in subjects prior to a cardiovascular surgical intervention and uses of a non-naturally occurring DNA sequence for performing these methods, as defined in the appended claims. The methods include detecting the expression level of the biomarker(s) p14ARF and / or p16INK4a in a blood sample obtained from a subject prior to the subject undergoing cardiovascular surgical intervention. The levels of these biomarkers are then compared to a pre-determined threshold of expression level. The subject is identified as being at risk of acute kidney injury when the expression level of the biomarker is equal to or higher than the pre-determined threshold level of expression.

Some embodiments described herein are methods for identifying risk of acute kidney injury in a subject comprising: obtaining a blood sample from a subject that will undergo or has undergone a surgical intervention or from a subject prior to or after being administered a chemotherapeutic. In some embodiments, the method comprises detecting the level of gene expression of p14^{ARF}. In some embodiments, the method comprises detecting the level of gene expression of p16^{INK4a}. In some embodiments, the method comprises detecting the level of gene expression of p16^{INK4a} and p14^{ARF}. In some embodiments, the method comprises detecting the level of gene expression of p14ARF, p16^{INK4a}, or p14^{ARF} and p16^{INK4a} in the sample; comparing the level of gene expression of p14^{ARF} or p16^{INK4a} or p14^{ARF} and p16^{INK4a} in the sample to a pre-determined threshold level of gene expression; and identifying the patient as being at risk of acute kidney injury when the gene expression level of at least one of p14^{ARF} and p16^{INK4a} is equal to or higher than the pre-determined threshold level of gene expression. In one aspect, the surgical intervention comprises a cardiovascular surgical intervention. In another aspect, the subject is identified as being susceptible to AKI prior to the surgical intervention. In another aspect, the subject is identified as being susceptible to AKI prior to recieving a chemotherapeutic agent.

Other instances described herein are methods of treating a patient identified as being at risk for developing AKI. In one aspect, the treatment comprises administering one or more prophylatic therapeutic regimens prior to a surgical intervention or prior to initiating chemotherapy. In another aspect, the treatment comprises administering a therapeutic regimen following surgical intervention or following initiating chemotherapy. In another aspect, the patient may already have AKI and the administered treatment reduces or minimizes the severity of AKI.

The methods described herein can be used to detect gene expression in a biological sample, and more particularly in a blood sample in a subject (e.g., a human patient). Gene expression levels can be determined in whole blood samples or, more typically, the whole blood sample can be manipulated or fractionated prior to determining gene expression level. Manipulation of blood samples is well known in the art and can include separation of red blood cells from white blood cells and plasma, or separation of various cell types from each other, including isolating specific white blood cells, or more specifically isolating T-lymphocytes, and measuring gene expression levels in the isolated cell type(s). In some embodiments, gene expression levels of p14^{ARF} and p16^{INK4a} are measured from a sample of isolated T-lymphocytes.

The level of gene expression can be determined using a variety of molecular biology techniques that are well known in the art. For example, if the expression level is to be determined by analyzing RNA isolated from the biological sample, techniques for determining the RNA expression level include, but are not limited to, Northern blotting, nuclease protection assays, quantitative PCR (e.g., digital RT-PCR and/or real time quantitative RT-PCR), branched DNA assay, direct sequencing of RNA by RNA seq, nCounter gene expression technology (NanoString Technologies), and nucleic acid microarrays. In some embodiments, expression levels are determined by real time quantitative PCR (RT-PCR) employing specific PCR primers for the p14^{ARF} and p16^{INK4a} genes. PCR primers for p14^{ARF} and p16^{INK4a} are described, for example, in US 8,158,347.

Alternatively, expression levels can be determined by analyzing protein levels in a biological sample using antibodies. Methods for quantifying specific proteins in biological samples are known in the art. Representative antibody-based techniques include, but are not limited to, immunodetection methods such as ELISA, Western blotting, in-cell Western, bead-based immunoaffinity, immunoaffinity columns, and 2-D gel separation.

Methods for nucleic acid isolation can comprise simultaneous isolation of total nucleic acid, or separate and/or sequential isolation of individual nucleic acid types (e.g., genomic DNA, cell-free RNA, organelle DNA, total cellular RNA, mRNA, polyA+ RNA, rRNA, tRNA) followed by optional combination of multiple nucleic acid types into a single sample. Such isolation techniques are known to those skilled in the art. Nucleic acids that are to be used for subsequent amplification and labeling can be analytically pure as determined by spectrophotometric measurements or by analysis following electrophoretic resolution (BioAnalyzer, Agilent). The nucleic acid sample can be free of contaminants such as polysaccharides, proteins, and inhibitors of enzyme reactions. When an RNA sample is intended for use as probe, it can be free of nuclease contamination. Contaminants and inhibitors can be removed or substantially reduced using resins for DNA extraction (e.g., CHELEX^{™} 100 from BioRad Laboratories, Hercules, Calif., United States of America) or by standard phenol extraction and ethanol precipitation. Isolated nucleic acids can optionally be fragmented by restriction enzyme digestion or shearing prior to amplification.

Various methods for designing primers for specific nucleic acid sequences of interest are well known in the art. Primers for amplifying p14^{ARF} and p16^{INK4a} separately can be designed based upon the specific sequences chosen. For example, p14^{ARF} and p16^{INK4a} transcripts each have a unique exon 1 but share a portion of exon 2. Therefore, to design primers specific for each of p14^{ARF} and p16^{INK4a}, a forward primer can be selected for each unique exon 1 and a reverse primer can be selected for the common exon 2. Conversely, suitable primers may be designed to amplify the shared portion of exon 2 of p14^{ARF} and p16^{INK4a} to determine the expression level of both genes together. In addition, it can be beneficial to design primers that flank the exon/intron junction, for example, to eliminate amplification signal from genomic DNA contamination in RT-PCR reaction.

In some embodiments of the present invention, the abundance of specific mRNA species present in a biological sample (for example, mRNA extracted from peripheral blood T lymphocytes) is assessed by quantitative RT-PCR. Standard molecular biological techniques are used in conjunction with specific PCR primers to quantitatively amplify those mRNA molecules corresponding to the gene or genes of interest. Methods for designing specific PCR primers and for performing quantitative amplification of nucleic acids including mRNA are well known in the art. See e.g., Heid et al., 1996; Sambrook & Russell, 2001; Joyce, 2002; Vandesompele et al., 2002. In some embodiments, a technique for determining expression level includes the use of the TAQMAN^{®} Real-time Quantitative PCR System (ThermoFisher Scientific, United States of America).

Specific primers for genes of interest (e.g., p16^{INK4a} and p14^{ARF}) are employed for determining expression levels of these genes. In some embodiments, the expression level of one or more housekeeping genes (e.g., 18S rRNA) are also determined in order to normalize a determined expression level. In some embodiments, the housekeeping gene is the YWHAZ gene. In one aspect, the level of expression of p16^{INK4a}and/or p14^{ARF} from a sample may be normalized to a house keeping gene from a batch of combined samples. In another aspect, the level of expression of p16^{INK4a} and/or p14^{ARF} from a sample may be normalized to a house keeping gene from the same sample.

The primers and probes used for amplification and detection may include a detectable label, such as a radiolabel, fluorescent label, or enzymatic label. *See,* U.S. Patent No US 5,869,717. In certain embodiments, the probe is fluorescently labeled. Fluorescently labeled nucleotides may be produced by various techniques, such as those described in Kambara et al., Bio/Technol., 6:816-21, (1988); Smith et al., Nucl. Acid Res., 13:2399-2412, (1985); and Smith et al., Nature, 321: 674-679, (1986).

The fluorescent dye may be linked to the deoxyribose by a linker arm that is easily cleaved by chemical or enzymatic means. There are numerous linkers and methods for attaching labels to nucleotides, as shown in Oligonucleotides and Analogues: A Practical Approach, IRL Press, Oxford, (1991); Zuckerman et al., Polynucleotides Res., 15: 5305-5321, (1987); Sharma et al., Polynucleotides Res.,19:3019, (1991); Giusti et al., PCR Methods and Applications, 2:223-227, (1993); Fung et al. (U.S. Patent Number 4,757,141); Stabinsky (U.S. Patent Number 4,739,044); Agrawal et al., Tetrahedron Letters, 31:1543-1546, (1990); Sproat et al., Polynucleotides Res., 15:4837, (1987); and Nelson et al., Polynucleotides Res., 17:7187-7194, (1989). Extensive guidance exists in the literature for derivatizing fluorophore and quencher molecules for covalent attachment via common reactive groups that may be added to a nucleotide. Many linking moieties and methods for attaching fluorophore moieties to nucleotides also exist, as described in Oligonucleotides and Analogues, supra; Guisti et al., *supra;* Agrawal et al., *supra;* and Sproat et al., *supra.*

The products of the Quantitative PCR employed in the TAQMAN^{®} Real-time Quantitative PCR System can be detected using a probe oligonucleotide that specifically hybridizes to the PCR product. Typically, this probe oligonucleotide is labeled at the 5' and/or 3' ends with one or more detectable labels described herein. In some embodiments, the 5' end is labeled with a fluorescent label and the 3' end is labeled with a fluorescence quencher. In some embodiments, the 5' end is labeled with tetrachloro-6-carboxyfluorescein (TET^{™}; Applera Corp., Norwalk, Conn., United States of America) and/or 6-FAM^{™} (Applera Corp.) and the 3' end includes a tetramethylrhodamine (TAMRA^{™}; Applera Corp.), NFQ, BHQ, and/or MGB quencher.

Additional exemplary and non-limiting detectable labels may be attached to the primer or probe and may be directly or indirectly detectable. The exact label may be selected based, at least in part, on the particular type of detection method used. Exemplary detection methods include radioactive detection, optical absorbance detection, e.g., UV-visible absorbance detection, optical emission detection, e.g., fluorescence; phosphorescence or chemiluminescence; Raman scattering. Preferred labels include optically-detectable labels, such as fluorescent labels. Examples of fluorescent labels include, but are not limited to, 4-acetamido-4'-isothiocyanatostilbene-2,2'disulfonic acid; acridine and derivatives: acridine, acridine isothiocyanate; 5-(2'-aminoethyl)aminonaphthalene-1-sulfonic acid (EDANS); 4-amino-N-[3-vinylsulfonyl)phenyllnaphthalimide-3,5 disulfonate; N-(4-anilino-1-naphthyl)maleimide; anthranilamide; BODIPY; alexa; fluorescin; conjugated multi-dyes; Brilliant Yellow; coumarin and derivatives; coumarin, 7-amino-4-methylcoumarin (AMC, Coumarin 120), 7-amino-4-trifluoromethylcouluarin (Coumaran 151); cyanine dyes; cyanosine; 4',6-diaminidino-2-phenylindole (DAPI); 5'5"-dibromopyrogallol-sulfonaphthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansylchloride); 4-dimethylaminophenylazophenyl-4'-isothiocyanate (DABITC); eosin and derivatives; eosin, eosin isothiocyanate, erythrosin and derivatives; erythrosin B, erythrosin, isothiocyanate; ethidium; fluorescein and derivatives; 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, QFITC, (XRITC); fluorescamine; IR144; IR1446; Malachite Green isothiocyanate; 4-methylumbelliferoneortho cresolphthalein; nitrotyrosine; pararosaniline; Phenol Red; B-phycoerythrin; o-phthaldialdehyde; pyrene and derivatives: pyrene, pyrene butyrate, succinimidyl 1-pyrene; butyrate quantum dots; Reactive Red 4 (Cibacron^{™} Brilliant Red 3B-A) rhodamine and derivatives: 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red); N,N,N',N'tetramethyl-6-carboxyrhodamine (TAMRA); tetramethyl rhodamine; tetramethyl rhodamine isothiocyanate (TRITC); riboflavin; rosolic acid; terbium chelate derivatives; Atto dyes, Cy3; Cy5; Cy5.5; Cy7; IRD 700; IRD 800; La Jolta Blue; phthalo cyanine; and naphthalo cyanine. Labels other than fluorescent labels are contemplated by the methods described herein, including other optically-detectable labels.

Other methodologies for determining gene expression levels can also be employed, including but not limited to Amplified Antisense RNA (aaRNA) and Global RNA Amplification (Van Gelder et al., 1990; Wang et al., 2000; U.S. Pat. No. 6,066,457 to Hampson et al.). In accordance with the methods of the presently disclosed subject matter, any one of the above-mentioned PCR techniques or related techniques can be employed to perform the step of amplifying the nucleic acid sample and/or quantitating the expression of a particular target nucleic acid. In addition, such methods can be optimized for amplification of a particular subset of nucleic acid (e.g., specific mRNA molecules versus total mRNA), and representative optimization criteria and related guidance can be found in the art. See Williams, 1989; Linz et al., 1990; Cha & Thilly, 1993; McPherson et al., 1995; Roux, 1995; Robertson & Walsh-Weller, 1998.

For any particular biomarker, graphical distributions of gene expression levels for subjects (e.g., preoperative subjects) that do or do not develop hospital acquired AKI following a cardiovascular surgical intervention are not completely distinct but instead will overlap. Therefore, any diagnostic test that measures a biomarker does not absolutely distinguish normal or low-risk patients from patients that are at risk for developing AKI with 100% accuracy. The graphical area of overlap correlates to a range of gene expression levels wherein the test cannot distinguish low-risk or normal from high risk. Thus, the developer of the test must select a threshold level of expression from the area of overlap and conclude that levels above the threshold are considered at risk for developing AKI and expression levels below the threshold are considered to be normal or not at risk. The smaller the area of overlap, the more accurate the diagnostic test will be.

Determining the exact threshold value to determine those that do or do not develop hospital acquired AKI (e.g., following a cardiovascular surgical intervention) will depend upon the assay format being developed. These threshold values may be determined empirically using techniques well known by those skilled in the art. For example, a threshold for determining a risk of acquiring AKI may be determined by obtaining a suitable biological sample from a population of patients in which a gene or gene product may be measured prior to undergoing surgery. In addition, a known identifier of post-operative AKI status, such as serum creatinine levels, is also measured to establish those patients that actually incurred post-operative AKI. Therefore, a useful population of patients will have a set of patients that incurred AKI and a set of pateints that did not incur AKI. The optimal threshold level for the assay may be determined by calculating the number of positively identified patients and negatively identified patients as having AKI at various gene expression threshold levels. The optimal threshold is a gene expression level that correctly identifies the highest percentage of patients as being at risk and not being at risk for developing AKI, thereby distinguishing two populations of patients.

One exemplary and non-limiting way to determine the ability of a particular test to distinguish two populations can be by using receiver operating characteristic (ROC) analysis. To draw a ROC curve, the true positive rate (TPR) and false positive rate (FPR) are determined as the decision threshold is varied continuously. Since TPR is directly correlated with sensitivity and FPR is inversely correlated with specificity (1-specificity), the ROC graph is sometimes called the sensitivity vs (1-specificity) plot. The area under the ROC curve is a measure of the probability that the perceived measurement will allow correct identification of a condition. A perfect test will have an area under the ROC curve of 1.0 whereas a random test will have an area of 0.5. Therefore, any actual diagnostic test analyzed using ROC analysis will have an area under the ROC curve somewhere between 0.5 and 1.0. The closer to 1.0 the curve is, the more accurate the test is.

ROC analysis is often used to select a threshold that provides an acceptable level of specificity and sensitivity to distinguish a "diseased" subpopulation from a "non-diseased" subpopulation. In general, optimal threshold is the point on the ROC curve closest to the upper left corner (100% sensitivity; 100% specificity). However, depending on the disease or patient population A more detailed description of ROC analysis and its use for evaluating diagnostic tests and predictive models can be found in the art, for example, in Zou et al., Circulation. 2007;115:654-657.

In addition to the measurement of AUC, the effectiveness of a given biomarker to predict or diagnose a disease can be estimated through several additional measures of diagnostic test accuracy (described in Fischer et al., Intensive Care Med. 29: 1043-51, 2003). These measures include sensitivity and specificity, likelihood ratios (LR), and diagnostic odds ratios (OR).

In one embodiment, the specificity of the assay for identifying AKI ranges from about 30% to about 100%, including each integer within the specified range. In one aspect, the specificity of the assay for identifying AKI ranges from about 50% to about 100%, including each integer within the specified range. In another aspect, the specificity of the assay for identifying AKI ranges from about 70% to about 100%, including each integer within the specified range. In another aspect, the specificity of the assay for identifying AKI ranges from about 30% to about 50%, including each integer within the specified range. In another aspect, the specificity of the assay for identifying AKI ranges from about 40% to about 60%, including each integer within the specified range. In another aspect, the specificity of the assay for identifying AKI ranges from about 50% to about 70%, including each integer within the specified range. In another aspect, the specificity of the assay for identifying AKI ranges from about 60% to about 80%, including each integer within the specified range. In another aspect, the specificity of the assay for identifying AKI ranges from about 70% to about 90%, including each integer within the specified range. In another aspect, the specificity of the assay is about 30%, about 35%, about 40%, about 45%, about 50%, about 55%, about 60%, about 65%, about 70%, about 75%, about 80%, about 85%, about 90%, about 95%, or even about 100%.

In another embodiment, the sensitivity of the assay for identifying AKI ranges from about 30% to about 100%, including each integer within the specified range. In one aspect, the sensitivity of the assay for identifying AKI ranges from about 50% to about 100%, including each integer within the specified range. In another aspect, the sensitivity of the assay for identifying AKI ranges from about 70% to about 100%, including each integer within the specified range. In another aspect, the sensitivity of the assay for identifying AKI ranges from about 30% to about 50%, including each integer within the specified range. In another aspect, the sensitivity of the assay for identifying AKI ranges from about 40% to about 60%, including each integer within the specified range. In another aspect, the sensitivity of the assay for identifying AKI ranges from about 50% to about 70%, including each integer within the specified range. In another aspect, the sensitivity of the assay for identifying AKI ranges from about 60% to about 80%, including each integer within the specified range. In another aspect, the sensitivity of the assay for identifying AKI ranges from about 70% to about 90%, including each integer within the specified range. In another aspect, the sensitivity of the assay is about 30%, about 35%, about 40%, about 45%, about 50%, about 55%, about 60%, about 65%, about 70%, about 75%, about 80%, about 85%, about 90%, about 95%, or even about 100%.

In another embodiment, the ROC curve area is an area ranging from about 0.5 to about 1, including each fractional integer within the specified range. In one aspect, the ROC curve area is greater than at least 0.5, at least 0.6, at least 0.7, at least 0.8, at least 0.9, or even at least 0.95.

In another embodiment, the suitable positive likelihood ratio is a ratio (calculated as sensitivity/(1-specificity)) of at least 1, at least 2, at least 3, at least 5, at least 10; and a negative likelihood ratio (calculated as (1-sensitivity)/specificity) of less than 1, less than or equal to 0.5, less than or equal to 0.3, less than or equal to 0.1; an odds ratio different from 1, at least about 2 or more, at least about 3 or more, at least about 4 or more, at least about 5 or more, or even at least about 10 or more.

In another embodiment, markers that predict AKI (i.e., levels of p14^{ARF} and/or p16^{INK4a}) can be coupled with other markers, e.g. markers of renal health- cystatin C, serum creatinine, NephroCheck^{®}, L-FABP. Methods for combining assay results can comprise the use of multivariate logistic regression, n-of-m analysis, decision tree analysis, calculating hazard ratios, etc. This list is not meant to be limiting. In these methods, a composite result which is determined by combining individual markers measured prior to intervention, maybe treated as if it itself a marker; that is, a threshold determined for composite result as described herein for individual markers, and the composite result can be used in to calculate odds ratio for individual patient. In one aspect, a subject is identified for being at risk of AKI by measuring the gene expression of p14^{ARF} and serum creatinine levels. In another aspect, a subject is identified for being at risk of AKI by measuring the gene expression of p16^{INK4a} and serum creatinine levels. In another aspect, a subject is identified for being at risk of AKI by measuring the gene expression of p14^{ARF} and p16^{INK4a} in addition to measuring serum creatinine levels.

In another embodiment, biomarkers can be used to stratify a subject population and identify a population where measurement of p14 and p16 predicts AKI and other adverse outcomes with the most sensitivity, specificity, and positive likelihood. These markers can be markers of organ function, inflammation status, etc or can be genetic markers. For example, a genetic marker for stratifying AKI subject populations is a single nucleotide polymorphism (SNP), which is located at chromosomal locus 9p21, specifically, rs10757278, rs2383206, rs2383207, or rs10757274. A mutation in both copies of each one of these loci is known to predispose patients to cardiovascular disease, *see,* for example U.S. Patent Application Publication No. US 2009/0150134. In addition, the measurement of p16 and/or p14 in patients with or without risk locus may require different thresholds.

Some embodiments described herein are non-naturally occurring DNA sequences, that are useful in identifying a subject as being at risk of developing AKI. These non-naturally occurring DNA sequences that are useful for establishing whether a subject is at risk of developing AKI contain at least one sequence segment that crosses at least one exon-exon boundary or untranslated region-exon boundary without containing the intervening intronic sequences. Therefore, these DNA sequences do not naturally occur. As would be understood by a person of ordinary skill, these non-naturally DNA sequences may be generated from a naturally occurring biological sample, such as RNA through reverse transcriptase-PCR followed by amplification with a suitable primer. In some aspects, the non-naturally occurring DNA sequence further comprises a non-natural or modified DNA base known by those skilled in the art.

Some embodiments described herein are methods of using the non-naturally occurring DNA sequences to identify subjects at risk for developing AKI. In one aspect, the non-naturally occurring DNA sequence is generated from a sample (e.g., a blood sample) from a subject and measured. Levels above a pre-determined threshold of the non-naturally occurring DNA sequence indicate a risk for developing AKI.

In some embodiments, the non-naturally occurring DNA sequences described herein comprise all or a portion of SEQ ID NO: 23 corresponding to the cDNA of the mRNA transcript of p16. In some aspects, the DNA sequence comprises between 10 and 1267 nucleotides of SEQ ID NO: 23, including each integer within the recited range. In some aspects, the DNA sequence comprises between 10 and 400 nucleotides of SEQ ID NO: 23, including each integer within the recited range. In some aspects, the DNA sequence comprises between 10 and 100 nucleotides of SEQ ID NO: 23, including each integer within the recited range. In some aspects, the DNA sequence comprises between 10 and 50 nucleotides of SEQ ID NO: 23, including each integer within the recited range. In some aspects, the non-naturally occurring DNA sequences comprise about 85% to about 100% homology to a portion or all of SEQ ID NO: 23. In some aspects, the non-naturally occurring DNA sequences comprise about 85%, about 90%, about 95%, about 99%, or 100% homology to a portion or all of SEQ ID NO: 23.

In some embodiments, the non-naturally occurring DNA sequences described herein comprises all or a portion of SEQ ID NO: 24 corresponding to the cDNA of the mRNA transcript of p14. In some aspects, the DNA sequence comprises between 10 and 1164 nucleotides of SEQ ID NO: 24, including each integer within the recited range. In some aspects, the DNA sequence comprises between 10 and 400 nucleotides of SEQ ID NO: 24, including each integer within the recited range. In some aspects, the DNA sequence comprises between 10 and 100 nucleotides of SEQ ID NO: 24, including each integer within the recited range. In some aspects, the DNA sequence comprises between 10 and 50 nucleotides of SEQ ID NO: 24, including each integer within the recited range. In some aspects, the non-naturally occurring DNA sequences comprise about 85% to about 100% homology to a portion or all of SEQ ID NO: 24. In some aspects, the non-naturally occurring DNA sequences comprise about 85%, about 90%, about 95%, about 99%, or 100% homology to a portion or all of SEQ ID NO: 24.

In some embodiments, the non-naturally occurring DNA sequences comprise the complimentary DNA sequence corresponding to a portion or the entire coding region of p16 (SEQ ID NO: 14). In some embodiments, the non-naturally occurring DNA sequences comprise the complimentary DNA sequence corresponding to a portion or the entire protein coding region of p14 (SEQ ID NO: 15). In some embodiments, the DNA sequences described herein may further contain a portion or all of the 5' untranslated region (UTR) of p16 (SEQ ID NO: 19). In some embodiments, the DNA sequences described herein may further contain a portion or all of the 3' untranslated region (UTR) of p16 (SEQ ID NO: 20). In some embodiments, the DNA sequences described herein may further contain a portion or all of the 5' untranslated region (UTR) of p14 (SEQ ID NO: 21). In some embodiments, the DNA sequences described herein may further contain a portion or all of the 3' untranslated region (UTR) of p14 (SEQ ID NO: 22).

In some embodiments, the non-naturally occurring DNA sequences comprise the complimentary DNA sequence corresponding to a portion or the entire sequence corresponding to SEQ ID NO: 29. In some embodiments, the non-naturally occurring DNA sequences comprise the complimentary DNA sequence corresponding to a portion or the entire sequence corresponding to SEQ ID NO: 30. In some embodiments, the non-naturally occurring DNA sequences comprise the complimentary DNA sequence corresponding to a portion or the entire sequence corresponding to SEQ ID NO: 31. In some embodiments, the non-naturally occurring DNA sequences comprise the complimentary DNA sequence corresponding to a portion or the entire sequence corresponding to SEQ ID NO: 32.

In one embodiment, the non-naturally occurring DNA sequence comprises a first DNA sequence segment, which is connected by a phosphodiester linkage to a second DNA sequence segment. In another embodiment, the first DNA sequence segment is connected by a phosphodiester linkage to a second DNA sequence and a third DNA sequence, such that the first DNA sequence is flanked by the second and third DNA sequences. In one aspect, the first DNA sequence comprises a portion or all of SEQ ID NO: 1. In another aspect, the second and third DNA sequences comprise a portion or all of any one of SEQ ID NO: 2-5.

In one aspect, the first DNA sequence segment has about an 88% identity to SEQ ID NO: 1. In another aspect, the first DNA sequence segment has a 100% identity to SEQ ID NO: 1.

In another aspect, the second DNA sequence segment comprises between 5 and 316 bases, including each integer thereof within the recited range, of anyone of SEQ ID NO: 2-5, including each integer within the recited range. In another aspect, the second DNA sequence segment comprises between 5 and 316 bases having at least a 50% identity to any one of SEQ ID NO: 2-5. In another aspect, the second DNA sequence segment comprises between 5 and 316 bases having at least a 55% identity to any one of SEQ ID NO: 2-5. In another aspect, the second DNA sequence segment comprises between 5 and 316 bases having at least a 60% identity to any one of SEQ ID NO: 2-5. In another aspect, the second DNA sequence segment comprises between 5 and 316 bases having at least a 65% identity to any one of SEQ ID NO: 2-5. In another aspect, the second DNA sequence segment comprises between 5 and 316 bases having at least a 70% identity to any one of SEQ ID NO: 2-5. In another aspect, the second DNA sequence segment comprises between 5 and 316 bases having at least a 75% identity to any one of SEQ ID NO: 2-5. In another aspect, the second DNA sequence segment comprises between 5 and 316 bases having at least a 80% identity to any one of SEQ ID NO: 2-5. In another aspect, the second DNA sequence segment comprises between 5 and 316 bases having at least a 85% identity to any one of SEQ ID NO: 2-5. In another aspect, the second DNA sequence segment comprises between 5 and 316 bases having at least a 90% identity to any one of SEQ ID NO: 2-5. In another aspect, the second DNA sequence segment comprises between 5 and 316 bases having at least a 95% identity to any one of SEQ ID NO: 2-5. In another aspect, the second DNA sequence segment comprises between 5 and 316 bases having at least a 97% identity to any one of SEQ ID NO: 2-5. In another aspect, the second DNA sequence segment comprises between 5 and 316 bases having at least a 98% identity to any one of SEQ ID NO: 2-5. In another aspect, the second DNA sequence segment comprises between 5 and 316 bases having at least a 99% identity to any one of SEQ ID NO: 2-5. In another aspect, the second DNA sequence segment comprises between 5 and 316 bases having a 100% identity to any one of SEQ ID NO: 2-5.

In another aspect, the third DNA sequence segment comprises between 5 and 316 bases having at least a 50% identity to any one of SEQ ID NO: 2-5, including each integer within the recited range. In another aspect, the third DNA sequence segment comprises between 5 and 316 bases having at least a 55% identity to any one of SEQ ID NO: 2-5. In another aspect, the third DNA sequence segment comprises between 5 and 316 bases having at least a 60% identity to any one of SEQ ID NO: 2-5. In another aspect, the third DNA sequence segment comprises between 5 and 316 bases having at least a 65% identity to any one of SEQ ID NO: 2-5. In another aspect, the third DNA sequence segment comprises between 5 and 316 bases having at least a 70% identity to any one of SEQ ID NO: 2-5. In another aspect, the third DNA sequence segment comprises between 5 and 316 bases having at least a 75% identity to any one of SEQ ID NO: 2-5. In another aspect, the third DNA sequence segment comprises between 5 and 316 bases having at least a 80% identity to any one of SEQ ID NO: 2-5. In another aspect, the third DNA sequence segment comprises between 5 and 316 bases having at least a 85% identity to any one of SEQ ID NO: 2-5. In another aspect, the third DNA sequence segment comprises between 5 and 316 bases having at least a 90% identity to any one of SEQ ID NO: 2-5. In another aspect, the third DNA sequence segment comprises between 5 and 316 bases having at least a 95% identity to any one of SEQ ID NO: 2-5. In another aspect, the third DNA sequence segment comprises between 5 and 316 bases having at least a 97% identity to any one of SEQ ID NO: 2-5. In another aspect, the third DNA sequence segment comprises between 5 and 316 bases having at least a 98% identity to any one of SEQ ID NO: 2-5. In another aspect, the third DNA sequence segment comprises between 5 and 316 bases having at least a 99% identity to any one of SEQ ID NO: 2-5. In another aspect, the third DNA sequence segment comprises between 5 and 316 bases having a 100% identity to any one of SEQ ID NO: 2-5.

In one embodiment, the non-naturally occurring DNA sequence comprises a portion or all of a first DNA sequence and a portion or all of a second DNA sequence segment. In one aspect, the non-naturally occurring DNA sequence comprises in a 5' to 3' direction a portion or all of SEQ ID NO: 2 connected by a phosphodiester linkage to a portion or all of SEQ ID NO: 1. In another aspect, the non-naturally occurring DNA sequence comprises in a 5' to 3' direction a portion or all of SEQ ID NO: 3 connected by a phosphodiester linkage to a portion or all of SEQ ID NO: 1. In another aspect, the non-naturally occurring DNA sequence comprises in a 5' to 3' direction a portion or all of SEQ ID NO: 1 connected by a phosphodiester linkage to a portion or all of SEQ ID NO: 4. In another aspect, the non-naturally occurring DNA sequence comprises in a 5' to 3' direction a portion or all of SEQ ID NO: 1 connected by a phosphodiester linkage to a portion or all of SEQ ID NO: 5. The non-naturally occurring DNA sequence may have an identity of 88% or 100% to SEQ ID NO: 1 and between a 50% and 100% identity to any one of SEQ ID NO: 2, 3, 4, and 5, as described herein, including each integer within the recited ranges.

In another embodiment, the non-naturally occurring DNA sequence comprises a first DNA sequence, a second DNA sequence segment, and a third DNA sequence segment. In one aspect, the non-naturally occurring DNA sequence comprises in a 5' to 3' direction a portion or all of SEQ ID NO: 2 connected by a phosphodiester linkage to a portion or all of SEQ ID NO: 1, wherein SEQ ID NO:1 is further connected by a phosphodiester linkage to a portion or all of SEQ ID NO: 4. In another aspect, the non-naturally occurring DNA sequence comprises in a 5' to 3' direction a portion or all of SEQ ID NO: 2 connected by a phosphodiester linkage to a portion or all of SEQ ID NO: 1, wherein SEQ ID NO:1 is further connected by a phosphodiester linkage to a portion or all of SEQ ID NO: 5. In another aspect, the non-naturally occurring DNA sequence comprises in a 5' to 3' direction a portion or all of SEQ ID NO: 3 connected by a phosphodiester linkage to a portion or all of SEQ ID NO: 1, wherein SEQ ID NO:1 is further connected by a phosphodiester linkage to a portion or all of SEQ ID NO: 4. The non-naturally occurring DNA sequence may have an identity of 88% or 100% to SEQ ID NO: 1 and between a 50% and 100% identity to any one of SEQ ID NO: 2, 3, 4, and 5, as described herein, including each integer within the recited range.

In another embodiment, the non-naturally occurring DNA sequence comprises a portion or all of SEQ ID NO: 6, 7, or 25. In one aspect, the non-naturally occurring DNA sequence has about a 50% sequence identity to SEQ ID NO: 6, 7, or 25. In another aspect, the non-naturally occurring DNA sequence has about a 60% sequence identity to SEQ ID NO: 6, 7, or 25. In another aspect, the non-naturally occurring DNA sequence has about a 70% sequence identity to SEQ ID NO: 6, 7, or 25. In another aspect, the non-naturally occurring DNA sequence has about a 80% sequence identity to SEQ ID NO: 6, 7, or 25. In another aspect, the non-naturally occurring DNA sequence has about a 85% sequence identity to SEQ ID NO: 6, 7, or 25. In another aspect, the non-naturally occurring DNA sequence has about a 90% sequence identity to SEQ ID NO: 6, 7, or 25. In another aspect, the non-naturally occurring DNA sequence has about a 95% sequence identity to SEQ ID NO: 6, 7, or 25. In another aspect, the non-naturally occurring DNA sequence has about a 97% sequence identity to SEQ ID NO: 6, 7, or 25. In another aspect, the non-naturally occurring DNA sequence has about a 99% sequence identity to SEQ ID NO: 6, 7, or 25. In another aspect, the non-naturally occurring DNA sequence has a 100% sequence identity to SEQ ID NO: 6, 7, or 25.

The non-naturally occurring DNA sequences described herein may comprise between 10 and 1,000 bases, including each integer within the specified range. In one aspect, the non-naturally occurring DNA sequence comprises between 10 and 500 bases, including each integer within the specified range. In another aspect, the non-naturally occurring DNA sequence comprises between 10 and 300 bases, including each integer within the specified range. In another aspect, the non-naturally occurring DNA sequence comprises between 10 and 200 bases, including each integer within the specified range. In another aspect, the non-naturally occurring DNA sequence comprises between 30 and 150 bases, including each integer within the specified range. In another aspect, the non-naturally occurring DNA sequence comprises between 30 and 75 bases, including each integer within the specified range.

Another embodiment described herein is a method for generating the non-naturally occurring DNA sequences described herein that are useful in identifying a subject at risk for developing AKI. First, a complimentary DNA (cDNA) sequence library may be generated by reverse transcription PCR (RT-PCR) from an RNA sample containing a pool of messenger RNA (mRNA). The RNA containing the mRNA may be purified from a sample (e.g., blood) by any suitable method known in the art and contacted with a suitable reverse transcriptase with non-specific primers. For example, suitable primers may contain poly deoxy-thymidine or may be randomized primers. This step generates the non-specific cDNA library. The non-naturally occurring DNA sequence may be generated by primers designed to specifically amplify the non-naturally occurring DNA sequence from the cDNA library. In one aspect, the primers for generating the non-naturally occurring DNA sequence comprises SEQ ID NO: 8, 9, 11, 12, 16, or 17, or combinations thereof. In one aspect, the primers for generating the non-naturally occurring DNA sequence comprises SEQ ID NO: 8 and SEQ ID NO: 9. In another aspect, the primers for generating the non-naturally occurring DNA sequence comprises SEQ ID NO: 11 and SEQ ID NO: 12. In another aspect, the primers for generating the non-naturally occurring DNA sequence comprises SEQ ID NO: 16 and SEQ ID NO: 17.

### Example 1

### Cardiac surgery clinical trial

Forty-seven patients were recruited into a prospective cohort study of older adults (median age 65.8 years) undergoing coronary artery bypass (CABG) surgical procedure. To be enrolled in the study, each patient must meet all of the inclusion criteria and none of the exclusion criteria. Inclusion criteria: 55 years of age and older undergoing primary elective or urgent coronary artery bypass surgery. Exclusion criteria: requiring emergency or salvage coronary artery bypass; re-operative procedure; undergoing combined aortic or valvular surgical procedures or primary ventricular assist device implantation; having any acute illness other than coronary artery disease; requiring preoperative inotropic or vasoactive medications. Venous blood samples were collected from each patient into an EDTA tube either during the patient's pre-operative visit to the clinic or intra-operatively after induction of general anesthesia but prior to surgical incision.

T cells were isolated from 6 ml of whole blood from each patient with RosetteSep^{™} Human T Cell Enrichment Cocktail (cat # 15061; Stemcell Technologies) using manufacturer's protocol and stored frozen at -80°C freezer. Total RNA was isolated from T cells using RNeasy Plus Mini Kit (cat# 74134; Qiagen) using manufacturer's protocol. RNA concentration was measured using NanoDrop 2000 spectrophotometer. cDNA was prepared from 1 µg of total RNA using ImProm-II reverse transcriptase (cat # A3801; Promega) and 0.5 µg of random primers (cat # C1181; Promega) using manufacturer's protocol. Resulting cDNA reactions were diluted 1:4 with distilled water. 4.5 ul of diluted cDNA was mixed with 5 ul of TaqMan 2x Universal PCR Master Mix (cat# 4324018; ThermoFisher Scientific) and 0.5 ul 20x Assay primer/probe mix (p16 primers: Forward 5'- CCAACGCACCGAATAGTTACG- 3'; Reverse 5'-GCGCTGCCCATCATCATG- 3'; p16 probe: 5' FAM-CCTGGATCGGCCTCCGAC-MGB NFQ - 3'; p14 primers: Forward 5'-CTGAGGAGCCAGCGTCTAG- 3'; Reverse 5'-CCCATCATCATGACCTGGTCTTCTA- 3'; p14 probe 5' FAM - CAGCAGCCGCTTCC-MGB NFQ- 3'; YWHAZ cat# 4331182 Hs03044281_g1; ThermoFisher Scientific); . Real-time PCR reactions were performed using ViiA7 PCR machine (Invitrogen). Cycle threshold (Ct) of 37 was used as a cutoff point and any expression signal ≥37 was disregarded. Expression of p16 or p14 was normalized using YWHAZ housekeeping gene (p16 ₙₒᵣₘ= p16ᵢ-YWHAZᵢ+YWHAZ_{average}). The p16 or p14 expression was calculated by subtracting normalized Ct values from 37 and the resulting values were used for ROC analysis. A threshold of 5.3 was selected for p16 and a threshold of 8.1 was selected for p14.

The absolute quantification of p16 and p14 gene transcripts in the biological samples was further quantified using a calibration standard. The calibration standard for determining p16 levels consisted of a p16 plasmid containing the open reading frame (ORF) of p16. Likewise, the calibration standard for determining p14 levels consisted of a p14 plasmid containing the open reading frame of p14. T7 RNA polymerase was used to in vitro transcribe reference complementary RNA (cRNA) from each of the p16 and p14 ORFs followed by quantification of the amount of cRNA (molecules/µl) in the sample. The reference cRNA and RNA samples from patients was reverse transcribed and quantitative RT-PCR was conducted and normalized to the YWHAZ gene from a Universal RNA. A standard curve was generated for the reference sample and the absolute number of transcripts was assessed by extrapolating the flourescence intensity values.

Peak serum creatinine after surgery was used to identify patients with AKI. Patients demonstrating an increase in serum creatinine of >=50% over baseline were identified as AKI positive whereas patient with a decrease, no change, or an increase of less than 50% were identified as AKI negative.

Of the 47 patients described in Example 1, 7 patients developed AKI and 40 patients did not (15% incidence). Demographics, eGFR and changes in creatinine (CRT) in patients that developed AKI are summarized in Table 1.

**Table 1.**

| Patient | Age | Gender | Race | BSL eGFR | Bsl CRT | Peak CRT | Increase in CRT |
|---|---|---|---|---|---|---|---|
| s057 | 81 | Female | White | >60 | 0.5 | 1 | 200% |
| s056 | 79 | Male | White | 45 | 1.5 | 4.7 | 313% |
| s010 | 79 | Male | White | >60 | 0.7 | 1.1 | 157% |
| s028 | 62 | Male | White | >60 | 0.9 | 1.4 | 156% |
| s008 | 69 | Male | White | >60 | 0.8 | 1.2 | 150% |
| s017 | 63 | Male | White | >60 | 1 | 1.8 | 180% |
| s027 | 61 | Male | White | >60 | 1.1 | 1.7 | 155% |

Expression levels of p16 and p14 and incidence of 30d major adverse clinical events (30d-MACE) in patients that developed AKI are summarized in Table 2.

**Table 2.**

| Patient | p16 PBTL | p14 PBTL | AKI | 30d-MACE | | | |
|---|---|---|---|---|---|---|---|
| | | | | Cardiac | Renal Failure | Infection Sternum | Other Adverse Outcomes |
| s057 | 6.82 | 8.72 | Yes | No | No | Yes | Discharged to rehab facility |
| s056 | 5.88 | 8.31 | Yes | Yes | Yes | No | Death |
| s010 | 5.96 | 8.25 | Yes | No | No | No | Remained in the ICU for 51h (33h median stay) |
| s028 | 5.39 | 8.24 | Yes | No | No | Yes | Readmitted to the hospital |
| s008 | 4.71 | 8.23 | Yes | No | No | No | |
| s017 | 4.41 | 8.21 | Yes | No | No | No | Discharged to rehab facility |
| s027 | 3.1 | 7.59 | Yes | No | No | No | |

As summarized in Table 3, out of 7 patients that developed AKI, only 1 would have been identified by eGFR<60 prior to surgery. Measurement of p16 would have identified 4 patients out of 7 who developed AKI while measurement of p 14 would have identified 6 patients out of 7 who developed AKI.

**Table 3.**

| | AKI Predicted By | | |
|---|---|---|---|
| Patient | p16 | p14 | eGFR |
| s057 | X | X | |
| s056 | X | X | X |
| s010 | X | X | |
| s028 | X | X | |
| s008 | | X | |
| s017 | | X | |
| s027 | | | |

Of the 40 patients that did not develop AKI, 22 had eGFR<60, 6 had p16 expression above the threshold of 5.3 and 9 had p14 expression above the threshold of 8.1.

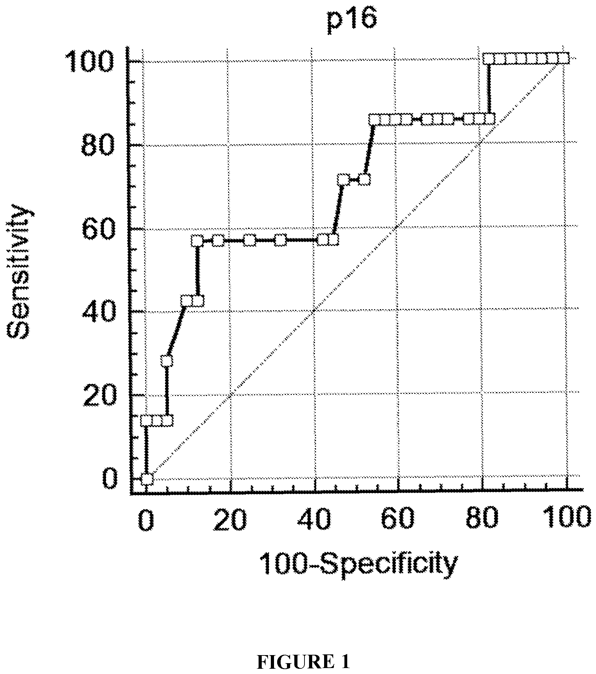

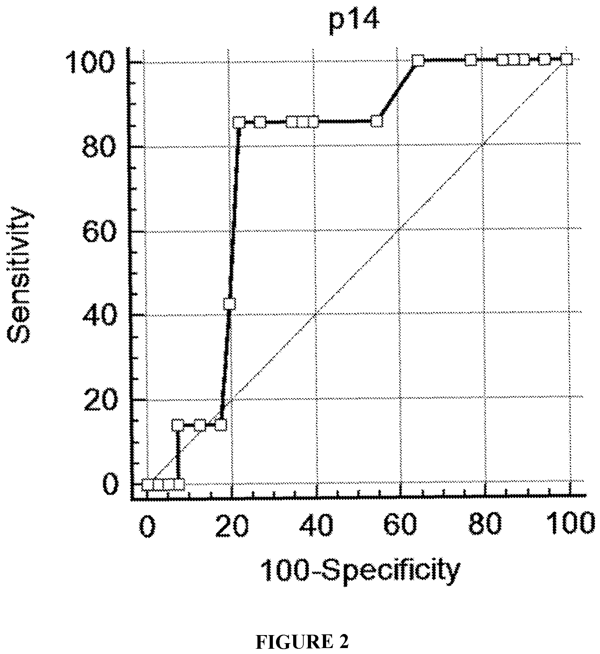

Receiver operating characteristic ("ROC") analysis of p16^{Ink4a} and p14^{Arf} expression levels in peripheral blood T lymphocytes (PBTL) in samples obtained from the patients described in Example I before coronary artery bypass grafting (CABG) surgery in which analysis was performed with respect to the clinical endpoint acute kidney injury (AKI) are shown in Figures 1-2.

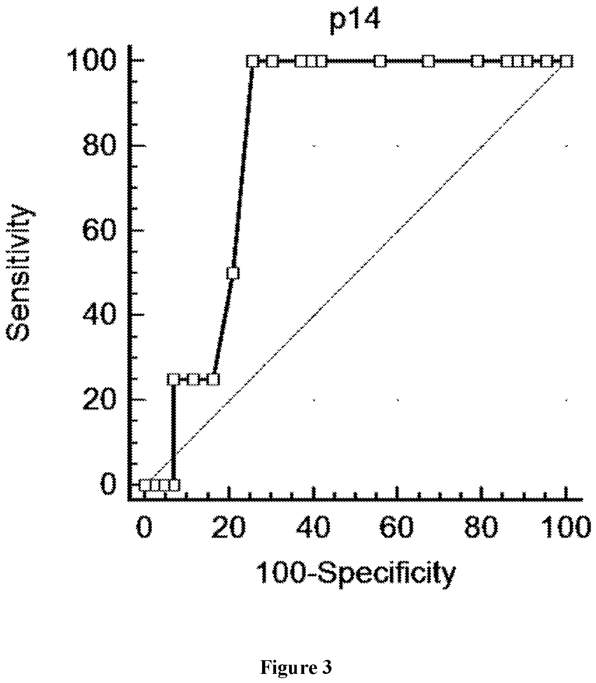

Receiver operating characteristic ("ROC") analysis of p14^{Arf} expression levels in peripheral blood T lymphocytes (PBTL) in samples obtained from the patients described in Example I before coronary artery bypass grafting (CABG) surgery in which analysis was performed with respect to 30-day MACE (major adverse clinical events: a composite of adverse outcomes, e.g. cardiac arrest, renal failure, deep sternum wound infection, sepsis, that lead to mortality or require recovery at a rehabilitation facility post surgery) are shown in Figure 3.

The parameters of diagnostic accuracy of expression of p16^{Ink4a} or p14^{Arf} to predict risk of developing AKI or a major adverse clinical event (30d-MACE) after cardiac artery bypass surgery are summarized in Table 4.

**Table 4**

| | p16INK4a_AKI | p14Arf_AKI | p14 Arf_30d MACE |
|---|---|---|---|
| AUC | 0.70 | 0.76 | 0.81 |
| Standard Error | 0.12 | 0.08 | 0.07 |
| p | 0.1 | 0.002 | <0.0001 |
| Sensitivity, % | 57 | 86 | 100 |
| Specificity, % | 88 | 78 | 74 |
| Positive likelihood ratio | 4.6 | 3.8 | 3.9 |
| Negative likelihood ratio | 0.5 | 0.2 | 0 |
| Odds Ratio | 6.1 | 15.8 | 25 |

Genomic DNA was isolated from 400 ul of whole blood from each patient using QIAamp DNA Blood Mini Plus Mini Kit (cat# 51104; Qiagen) using manufacturer's protocol. DNA concentration was measured using NanoDrop 2000 spectrophotometer. SNP status was determined by real-time PCR using commercial, pre-designed TaqMan^{®} SNP Genotyping Assays (ThermoFisher Scientific). 150ng of diluted genomic DNA (15 ul volume) was mixed with 2.5 ul of TaqMan Genotyping Master Mix (cat# 4371353; ThermoFisher Scientific) and 0.25 ul 40x TaqMan SNP Genotyping Assay (C_11841860_10 for rs10757278; C_1754669_10 for rs2383206; C_15789010_10 for rs2383207; C_26505812_10 for, cat # 4351379 ThermoFisher Scientific). Real-time PCR reactions were performed using ViiA7 PCR machine (Invitrogen). Single nucleotide polymorphism status was reported by manufacture's software as AA, AG or GG.

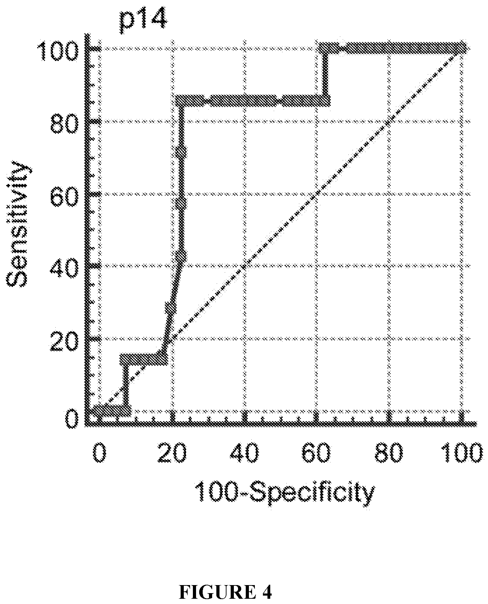

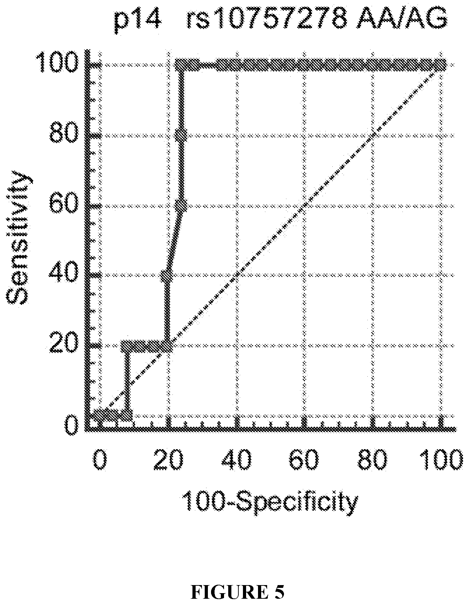

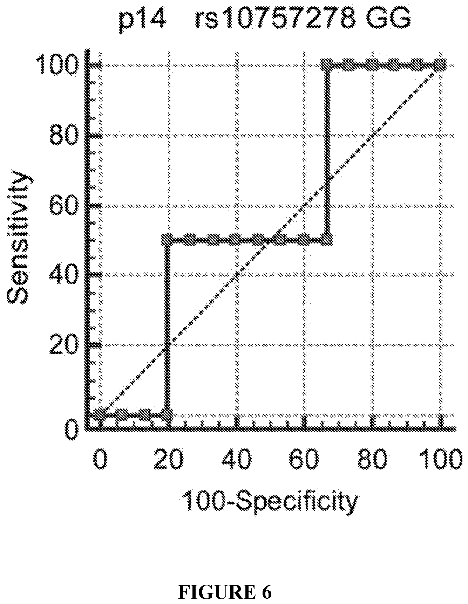

Receiver operating characteristic ("ROC") analysis of p14^{Arf} expression levels in peripheral blood T lymphocytes (PBTL) in samples obtained from the patients described in Example I before coronary artery bypass grafting (CABG) surgery in which analysis was performed with respect to the clinical endpoint acute kidney injury (AKI) are shown in Figures 4-6. Patients in the study were stratified into two groups, those with wild-type or heterozygous rs10757278 (AA or AG) and those with homozygous, mutated rs10757278 (GG; risk locus).

The parameters of diagnostic accuracy of expression of p14^{Arf} to predict risk of developing AKI after cardiac surgery in patients from the entire study or population that excluded risk locus rs10757278 patients are summarized in Table 5. A threshold of 8.1 was selected for p14 in both populations.

**Table 5**

| rs10757278 | Entire study | AA/AG |
|---|---|---|
| | p14Arf_AKI | p14Arf_AKI |
| AUC | 0.75 | 0.80 |
| Standard Error | 0.09 | 0.08 |
| p | 0.004 | 0.0001 |
| Sensitivity, % | 86 | 100 |
| Specificity, % | 78 | 76 |
| Positive likelihood ratio | 3.8 | 4.2 |
| Negative likelihood ratio | 0.2 | 0 |
| Odds Ratio | 15.8 | 33 |

### Example 2.

### Catheterization Study

Two hundred patients undergoing cardiac catheterization with or without percutaneous coronary intervention (PCI) are recruited in the study. To be enrolled in the study, each patient must meet all of the inclusion criteria and none of the exclusion criteria. Inclusion criteria: 18 years of age or older and have at least one risk factor that places them at moderate risk for kidney injury (≥14% as defined by Mehran et al., J. Am. Coll. Cardiol. 44(7) pp. 1393-1399 (2004). PMID: 15464318). Patients that fall into that group could have congestive heart disease stage III/IV (as defined by the New York Heart Association) or chronic kidney disease (15<eGFR<60 ml/min/1.73 m²) and diabetes or age >75 with either one of above condition. Exclusion criteria: have of acute, active infection (e.g. HIV, pneumonia, septic shock); contrast media exposure within last 72h; presenting with STEMI; presence of cardiogenic shock; presence of hemodynamic instability or requiring pressors of IABP; severe heart failure with known EF<25%; heart transplant patient or LVAD patient; receiving dialysis for end stage renal disease; kidney transplant and liver transplant patients and all patients currently on immunosuppressants; chronic liver disease /cirrhosis; history of cancer and chemotherapy except basal cell carcinoma or squamous skin cancer.

Venous blood is collected into an EDTA tube from each patient prior to catheterization procedure. Peripheral blood T lymphocytes and peripheral blood mononucleated cells are then isolated form fresh blood and stored frozen for analysis of p16 and p14Arf expression. Peripheral blood is collected at 10-20h from patients undergoing stenting and at 48-72 from all patients for measurement of markers of kidney function. Urine is collected at baseline and at 10-20h from patients undergoing stenting for measurement of markers of early kidney injury.

Change in serum creatinine at 48-72h as compared to baseline is used to identify patients with AKI (increase in serum creatinine of >=25% over baseline).

Change in other plasma and urine biomarkers (Cystatin C, NGAL, KIM-1, L-FABP) between 10-20h post procedure and baseline are used to provide alternative methods of identifying patients with AKI.

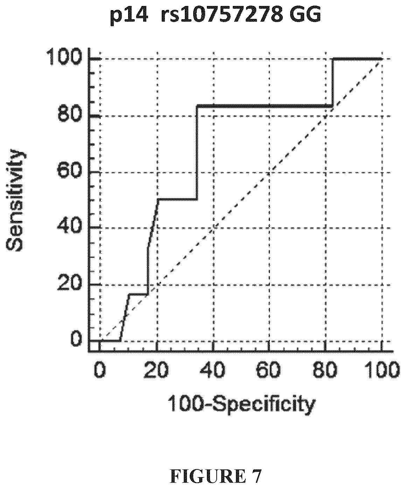

Similar to Example 1, expression of p16 and p14 is measured in T lymphocytes and used in receiver operating curve (ROC) analysis to determine threshold, sensitivity, specificity, and AUC for discriminating between non-diseased and diseased (AKI) populations (Figure 7).

| **Table 6. Example 2 Statistical Parameters** | |
|---|---|
| | p14_AKI |
| AUC | 0.67 |
| Standard Error | 0.12 |
| p | 0.16 |
| Sensitivity, % | 83 |
| Specificity, % | 66 |
| Positive likelihood ratio | 2.42 |
| Negative likelihood ratio | 0.25 |

### Example 3

### Cardiac Surgery Clinical Trial

186 patients are recruited into a prospective cohort study of older adults undergoing coronary artery bypass (CABG) surgical procedure at Duke University Hospitals. To be enrolled in the study, each patient must meet all of the inclusion criteria and none of the exclusion criteria. Inclusion criteria: 40 years of age and older undergoing non-emergency cardiac surgery using cardiopulmonary bypass (CABG, combined CABG/valve, or valve surgery). Exclusion criteria: requiring emergency surgery; off-pump coronary bypass grafting, aortic aneurysm repair; congenital heart disease repair; heart transplant or LVAD patient; severe heart failure (LVEF<25%); hemodynamic instability or requiring preoperative vasopressors or IABP; pre-existing end-stage kidney disease or renal transplantation; presence of major active infection; or chronic liver disease/cirrhosis. Venous blood samples were collected from each patient into an EDTA tube either during the patient's pre-operative visit to the clinic or intra-operatively after induction of general anesthesia but prior to surgical incision.

T cells were isolated from 5 ml of whole blood from each patient with RosetteSep^{™} Human T Cell Enrichment Cocktail (cat# 15061; Stemcell Technologies), using the manufacturer's protocol, and stored frozen at -80°C. Total RNA was isolated from T cells using the Quick-RNA MiniPrep kit (cat# R1055; Zymo Research) using the manufacturer's protocol. RNA concentration was measured using a NanoDrop 8000 spectrophotometer. cDNA was prepared from 400 ng of total RNA using ImProm-II reverse transcriptase (cat# A3801; Promega) and 0.5µg of random hexamers (Eton Bioscience) using the manufacturer's protocol. Resulting cDNA reactions were diluted 1:4 with distilled water. 4.5 ul of diluted cDNA was mixed with 5 ul of iTaq Universal Probes Supermix (cat# 1725134; Bio-Rad) and 0.5 ul 20x Assay primer/probe mix (p16^{INK4a} primers: Forward 5'-CCAACGCACCGAATAGTTACG-3'; Reverse 5'- GCGCTGCCCATCATCATG- 3'; p16^{INK4a} probe: 5' FAM-CCTGGATCGGCCTCCGAC-MGB NFQ-3'; p14^{ARF} primers: Forward 5'-CTCGTGCTGATGCTACTGA-3'; Reverse 5'-TCATCATGACCTGGTCTTCT-3'; p14^{ARF} probe 5'-[6-FAM]AAGCGGCTGCTGCCCTAGAC[BHQ1a-Q]-3'; YWHAZ cat# 4331182 = Hs03044281_g1, ThermoFisher Scientific;). Real-time PCR reactions were performed using the CFX384 Touch real-time PCR detection system (Bio-Rad). Cycle threshold (Ct) of 37 was used as a cutoff point and any expression signal ≥37 was disregarded. Expression of p16^{INK4a} or p14^{ARF} was normalized using the YWHAZ housekeeping gene (p16^{INK4a} norm = p16^{INK4a} i - YWHAZi). p16^{INK4a} or p14^{ARF} expression was calculated by subtracting normalized Ct values from 20 and the resulting values were used for ROC analysis.

Peak serum creatinine after surgery was used to identify patients with AKI. Patients demonstrating an increase in serum creatinine of 0.3 mg/dL or >=50% over baseline within 7 days of surgery were identified as AKI positive whereas patient with a decrease, no change, or an increase of less than 0.3 mg/dL or 50% were identified as AKI negative.

Genomic DNA was isolated from 100 ul of whole blood from each patient using Quick-DNA 96 kit (cat# D3011; Zymo Research) using the manufacturer's protocol. DNA concentration was measured using a NanoDrop 8000 spectrophotometer. SNP status was determined by real-time PCR using commercial, pre-designed TaqMan^{®} SNP Genotyping Assays (ThermoFisher Scientific). 40 ng of diluted genomic DNA (4 ul volume) was mixed with 5 ul of iTaq Universal Probes Supermix (cat# 1725134; Bio-Rad) and 0.25 ul 40x TaqMan SNP Genotyping Assay (C_11841860_10 for rs10757278; C_1754669_10 for rs2383206; C_15789010_10 for rs2383207; C_26505812_10 for rs10757274; all cat # 4351379, ThermoFisher Scientific). Real-time PCR reactions were performed using CFX384 Touch real-time PCR detection system (Bio-Rad). Single nucleotide polymorphism status was reported by manufacture's software as AA (allele 1), AG (heterozygote) or GG (allele 2). The results of the study are not shown but are consistent with the data of Example 1.

## Claims

1. A method for identifying risk of acute kidney injury in subjects prior to cardiovascular surgical intervention comprising:
a) detecting a level of gene expression of p14^{ARF} or p16^{INK4a} or p14^{ARF} and p16^{INK4a} in a blood sample obtained from a subject prior to the subject undergoing cardiovascular surgical intervention; and
b) comparing the level of gene expression of p14^{ARF} or p16^{INK4a} or p14^{ARF} and p16^{INK4a} in the sample to a pre-determined threshold level of gene expression of p14^{ARF} or p16^{INK4a} or p14^{ARF} and p16^{INK4a}; wherein the subject is identified as being at risk of acute kidney injury when the level of gene expression of at least one of or both of p14^{ARF} or p16^{INK4a} is equal to or higher than the pre-determined threshold level of gene expression.

2. The method of claim 1, further comprising isolating peripheral blood T lymphocytes from the blood sample of step (a), and detecting the level of expression of p14^{ARF} or p16^{INK4a} or p14^{ARF} and p16^{INK4a} in the peripheral blood T lymphocytes from the sample.

3. The method according to claims 1 or 2, wherein the level of gene expression is detected by a method comprising quantitative PCR, microarray, or RNA sequencing.

4. The method according to any one of claims 1-3, wherein the level of gene expression is detected by detecting a gene product of p14^{ARF}or p16^{INK4a} or p14^{ARF} and p16^{INK4a} in the sample.

5. The method according to any one of claim 1-4, wherein the level of gene expression is detected by generating a non-naturally occurring DNA sequence from the blood sample from the subject, wherein the sequence comprises a first DNA sequence segment having 88% identity to SEQ ID NO: 1 connected by a phosphodiester linkage to a second DNA sequence segment comprising between 5 and 316 bases having 85% identity to one or more of SEQ ID NO: 2-5.

6. The method according to claim 5, wherein the non-naturally occurring sequence comprises the first DNA sequence segment that is connected by a phosphodiester linkage to a third DNA sequence segment comprising between 5 and 316 bases having 85% identity to any one of SEQ ID NO: 2-5.

7. The method according to claim 5, wherein the non-naturally occurring DNA sequence comprises a portion of or all of SEQ ID NO: 2, SEQ ID NO: 1, and SEQ ID NO: 4 in a 5' to 3' direction or wherein the non-naturally occurring DNA sequence comprises a portion of or all of SEQ ID NO: 3, SEQ ID NO: 1, and SEQ ID NO: 4 in a 5' to 3' direction.

8. The method according to claim 5, wherein the sequence comprises SEQ ID NO: 6, 7, or 25 or combinations thereof.

9. The method according to any one of claims 5-8, wherein a total combined sequence length comprises between 30 and 150 bases or between 30 and 75 bases.

10. The method according to any one of claims 5-9, wherein the pre-determined threshold is determined by ROC analysis.

11. The method according to any one of claims 5-10, wherein the cardiovascular surgical intervention comprises a cardiac catheterization procedure.

12. Use of a non-naturally occurring DNA sequence for performing the method of any one of claims 1-11 comprising:
a) generating the non-naturally occurring DNA sequence from a blood sample obtained from the subject, wherein the sequence comprises a first DNA sequence segment having 88% identity to SEQ ID NO: 1 connected by a phosphodiester linkage to a second DNA sequence segment comprising between 5 and 316 bases having 85% identity to one or more of SEQ ID NO: 2-5;
b) detecting a level of the non-naturally occurring DNA sequence;
c) comparing the level of the non-naturally occurring DNA sequence to a pre-determined threshold level of the non-naturally occurring DNA sequence; and
d) identifying the subject as being at risk of acute kidney injury when the level of the non-naturally occurring DNA sequence is equal to or higher than a pre-determined threshold level of the non-naturally occurring DNA sequence.

## Patentansprüche

1. Verfahren zur Identifizierung des Risikos einer akuten Nierenverletzung bei Subjekten vor einem kardiovaskulären chirurgischen Eingriff, umfassend:
a) Detektieren eines Genexpressionsniveaus von p14^{ARF} oder p16^{INK4a} oder p14^{ARF} und p16^{INK4a} in einer Blutprobe, die von einem Subjekt erhalten wurde, bevor sich das Subjekt einem kardiovaskulären chirurgischen Eingriff unterzieht; und
b) Vergleichen des Genexpressionsniveaus von p14^{ARF} oder p16^{INK4a} oder p14^{ARF} und p16^{INK4a} in der Probe mit einem vorbestimmten Schwellenwert der Genexpression von p14^{ARF} oder p16^{INK4a} oder p14^{ARF} und p16^{INK4a}; wobei das Subjekt als Risikosubjekt für eine akute Nierenverletzung identifiziert wird, wenn das Genexpressionsniveau von mindestens einem oder beiden von p14^{ARF} oder p16^{INK4a} gleich oder höher als der vorbestimmte Schwellenwert der Genexpression ist.

2. Verfahren gemäß Anspruch 1, ferner umfassend die Isolierung von T-Lymphozyten aus peripherem Blut aus der Blutprobe von Schritt (a), und das Nachweisen des Expressionsniveaus von p14^{ARF} oder p16^{INK4a} oder p14^{ARF} und p16^{INK4a} in den T-Lymphozyten des peripheren Blutes aus der Probe.

3. Verfahren gemäß irgendeinem der Ansprüche 1 oder 2, wobei das Genexpressionsniveau durch ein Verfahren detektiert wird, das quantitative PCR, Microarray oder RNA-Sequenzierung umfasst.

4. Verfahren gemäß irgendeinem der Ansprüche 1 bis 3, wobei das Genexpressionsniveau durch Detektieren eines Genprodukts von p14^{ARF} oder p16^{INK4a} oder p14^{ARF} und p16^{INK4a} in der Probe detektiert wird.

5. Verfahren gemäß irgendeinem der Ansprüche 1-4, wobei das Genexpressionsniveau durch Erzeugen einer nicht natürlich vorkommenden DNA-Sequenz aus der Blutprobe des Subjekts detektiert wird, wobei die Sequenz ein erstes DNA-Sequenzsegment mit 88% Identität mit SEQ ID NO: 1 umfasst, das durch eine Phosphodiesterbindung mit einem zweiten DNA-Sequenzsegment verbunden ist, das zwischen 5 und 316 Basen umfasst und 85% Identität mit einer oder mehreren der SEQ ID NO: 2-5 aufweist.

6. Verfahren gemäß Anspruch 5, wobei die nicht natürlich vorkommende Sequenz das erste DNA-Sequenzsegment umfasst, das durch eine Phosphodiesterbindung mit einem dritten DNA-Sequenzsegment verbunden ist, das zwischen 5 und 316 Basen mit 85% Identität zu einer der SEQ ID NO: 2-5 umfasst.

7. Verfahren gemäß Anspruch 5, wobei die nicht-natürlich vorkommende DNA-Sequenz einen Teil oder die Gesamtheit von SEQ ID NO: 2, SEQ ID NO: 1 und SEQ ID NO: 4 in einer 5' zu 3'-Richtung umfasst, oder wobei die nicht natürlich vorkommende DNA-Sequenz einen Teil oder die Gesamtheit von SEQ ID NO: 3, SEQ ID NO: 1 und SEQ ID NO: 4 in einer 5'- zu 3'-Richtung Richtung umfasst.

8. Verfahren gemäß Anspruch 5, wobei die Sequenz SEQ ID NO: 6, 7, oder 25 oder Kombinationen davon umfasst.

9. Verfahren gemäß irgendeinem der Ansprüche 5-8, wobei eine gesamte kombinierte Sequenzlänge zwischen 30 und 150 Basen oder zwischen 30 und 75 Basen umfasst.

10. Verfahren gemäß irgendeinem der Ansprüche 5-9, wobei der vorbestimmte Schwellenwert durch ROC-Analyse bestimmt wird.

11. Verfahren gemäß irgendeinem der Ansprüche 5-10, wobei der kardiovaskuläre chirurgische Eingriff eine Herzkatheter Prozedur umfasst.

12. Verwendung einer nicht natürlich vorkommenden DNA-Sequenz zur Durchführung des Verfahrens gemäß irgendeinem der Ansprüche 1-11, umfassend:
a) Erzeugen der nicht natürlich vorkommenden DNA-Sequenz aus einer von dem Subjekt erhaltenen Blutprobe, wobei die Sequenz ein erstes DNA-Sequenzsegment mit 88% Identität mit SEQ ID NO: 1 umfasst, das durch eine Phosphodiesterbindung mit einem zweiten DNA-Sequenzsegment verbunden ist, das zwischen 5 und 316 Basen umfasst und 85% Identität mit einer oder mehreren von SEQ ID NO: 2-5 aufweist;
b) Detektieren eines Niveaus der nicht natürlich vorkommenden DNA-Sequenz;
c) Vergleichen des Niveaus der nicht natürlich vorkommenden DNA-Sequenz mit einem vorbestimmten Schwellenwert der nicht natürlich vorkommenden DNA-Sequenz; und
d) Identifizieren des Subjekts als Risikosubjekt für eine akute Nierenverletzung, wenn das Niveau der nicht natürlich vorkommenden DNA-Sequenz gleich oder höher ist als ein vorbestimmter Schwellenwert der nicht natürlich vorkommenden DNA-Sequenz.

## Revendications

1. Procédé d'identification du risque de lésion rénale aiguë chez des sujets avant une intervention chirurgicale cardiovasculaire comprenant les étapes consistant à :
a) détecter un niveau d'expression génique de p14^{ARF} ou de p16^{INK4a} ou de p14^{ARF} et de p16^{INK4a} dans un échantillon de sang prélevé sur un sujet avant que le sujet subisse une intervention chirurgicale cardiovasculaire ; et
b) comparer le niveau d'expression génique de p14^{ARF} ou de p16^{INK4a} ou de p14^{ARF} et de p16^{INK4a} dans l'échantillon à un niveau seuil prédéterminé d'expression génique de p14^{ARF} ou de p16^{INK4a} ou de p14^{ARF} et de p16^{INK4a} ; dans lequel le sujet est identifié comme étant à risque de lésion rénale aiguë quand le niveau d'expression génique d'au moins l'un ou des deux parmi p14^{ARF} ou p16^{INK4a} est supérieur ou égal au niveau seuil prédéterminé d'expression génique.

2. Procédé selon la revendication 1, comprenant en outre l'isolement de lymphocytes T de sang périphérique à partir de l'échantillon de sang de l'étape (a), et la détection du niveau d'expression de p14^{ARF} ou de p16^{INK4a} ou de p14^{ARF} et de p16^{INK4a} dans les lymphocytes T de sang périphérique provenant de l'échantillon.

3. Procédé selon les revendications 1 ou 2, dans lequel le niveau d'expression génique est détecté par un procédé comprenant une PCR quantitative, un microréseau, ou un séquençage d'ARN.

4. Procédé selon l'une quelconque des revendications 1-3, dans lequel le niveau d'expression génique est détecté par la détection d'un produit génique de p14^{ARF}ou de p16^{INK4a} ou de p14^{ARF} et de p16^{INK4a} dans l'échantillon.

5. Procédé selon l'une quelconque des revendications 1-4, dans lequel le niveau d'expression génique est détecté en générant une séquence d'ADN non naturelle à partir de l'échantillon de sang provenant du sujet, dans lequel la séquence comprend un premier segment de séquence d'ADN présentant 88 % d'identité avec SEQ ID NO : 1 relié par une liaison phosphodiester à un deuxième segment de séquence d'ADN comprenant entre 5 et 316 bases présentant 85 % d'identité avec une ou plusieurs parmi SEQ ID NO : 2-5.

6. Procédé selon la revendication 5, dans lequel la séquence non naturelle comprend le premier segment de séquence d'ADN qui est relié par une liaison phosphodiester à un troisième segment de séquence d'ADN comprenant entre 5 et 316 bases présentant 85 % d'identité avec l'une quelconque parmi SEQ ID NO : 2-5.

7. Procédé selon la revendication 5, dans lequel la séquence d'ADN non naturelle comprend une partie ou la totalité de SEQ ID NO : 2, SEQ ID NO : 1, et SEQ ID NO : 4 dans une direction 5' vers 3' ou dans lequel la séquence d'ADN non naturelle comprend une partie ou la totalité de SEQ ID NO : 3, SEQ ID NO : 1, et SEQ ID NO : 4 dans une direction 5' vers 3'.

8. Procédé selon la revendication 5, dans lequel la séquence comprend SEQ ID NO : 6, 7, ou 25 ou des combinaisons de celles-ci.

9. Procédé selon l'une quelconque des revendications 5-8, dans lequel une longueur totale de séquence combinée comprend entre 30 et 150 bases ou entre 30 et 75 bases.

10. Procédé selon l'une quelconque des revendications 5-9, dans lequel le seuil prédéterminé est déterminé par une analyse ROC.

11. Procédé selon l'une quelconque des revendications 5-10, dans lequel l'intervention chirurgicale cardiovasculaire comprend une procédure de cathétérisme cardiaque.

12. Utilisation d'une séquence d'ADN non naturelle pour mettre en oeuvre le procédé selon l'une quelconque des revendications 1-11 comprenant les étapes consistant à :
a) générer la séquence d'ADN non naturelle à partir d'un échantillon de sang prélevé sur le sujet, dans lequel la séquence comprend un premier segment de séquence d'ADN présentant 88 % d'identité avec SEQ ID NO : 1 relié par une liaison phosphodiester à un deuxième segment de séquence d'ADN comprenant entre 5 et 316 bases présentant 85 % d'identité avec une ou plusieurs parmi SEQ ID NO : 2-5 ;
b) détecter un taux de la séquence d'ADN non naturelle ;
c) comparer le taux de la séquence d'ADN non naturelle à un taux seuil prédéterminé de la séquence d'ADN non naturelle ; et
d) identifier le sujet comme étant à risque d'une lésion rénale aiguë quand le taux de la séquence d'ADN non naturelle est supérieur ou égal à un taux seuil prédéterminé de la séquence d'ADN non naturelle.
